(19) Europäisches Patentamt
European Patent Office
Office européen des brevets

(11) **EP 4 609 881 A1**

(12) **EUROPEAN PATENT APPLICATION**
published in accordance with Art. 153(4) EPC

(43) Date of publication:
**03.09.2025 Bulletin 2025/36**

(21) Application number: **23882692.9**

(22) Date of filing: **25.10.2023**

(51) International Patent Classification (IPC):
***A61K 47/68*** (2017.01) ***A61K 9/08*** (2006.01)
***A61K 39/395*** (2006.01) ***A61K 47/18*** (2017.01)
***A61K 47/26*** (2006.01) ***A61K 51/10*** (2006.01)
***A61P 35/00*** (2006.01) ***C07K 16/18*** (2006.01)

(52) Cooperative Patent Classification (CPC):
**A61K 9/08; A61K 39/395; A61K 47/18; A61K 47/26; A61K 47/68; A61K 51/10; A61P 35/00; C07K 16/18**

(86) International application number:
**PCT/JP2023/038580**

(87) International publication number:
**WO 2024/090488 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.10.2022 JP 2022171832**

(71) Applicant: **Nihon Medi-Physics Co., Ltd.**
**Tokyo 136-0075 (JP)**

(72) Inventors:
- **TAKEMORI, Hideaki**
  **Tokyo 136-0075 (JP)**
- **OKUMURA, Yuki**
  **Tokyo 136-0075 (JP)**
- **TAKENAKA, Fumiaki**
  **Tokyo 136-0075 (JP)**
- **TONOYA, Gota**
  **Tokyo 136-0075 (JP)**
- **KAWAMURA, Eriko**
  **Suita-shi, Osaka 564-0053 (JP)**
- **HORIUCHI, Shohei**
  **Suita-shi, Osaka 564-0053 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **RADIOACTIVE PHARMACEUTICAL COMPOSITION**

(57) The present invention relates to a liquid pharmaceutical composition containing a conjugate of a radionuclide and an antibody as an active ingredient. It is characterized in that the antibody is an antibody that specifically binds to mucin subtype 5AC, the concentration of the aforementioned antibody in the composition is 0.1 mg/mL or more and 5 mg/mL or less, and the composition contains L-arginine, L-histidine, and a surfactant. Such pharmaceutical composition shows reduced radioactivity adsorption to a storage container.

[Fig. 2]

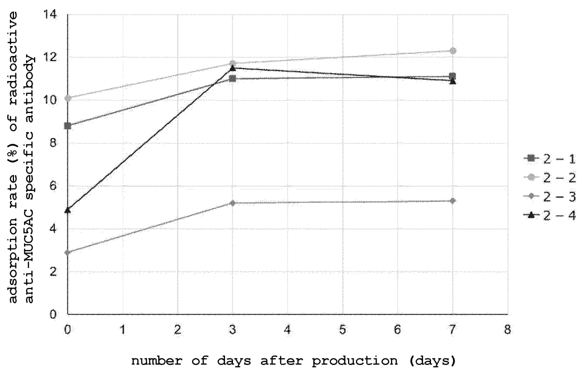


EP 4 609 881 A1

## Description

[Technical Field]

The present invention relates to a liquid radioactive pharmaceutical composition, particularly a liquid pharmaceutical composition containing a conjugate of a radionuclide and an antibody that specifically binds to mucin subtype 5AC as an active ingredient.

[Background Art]

Mucin is the main component of mucus secreted from epithelial cell and the like of animal and is a glycoprotein with a molecular weight of 1 - 10 million containing a large amount of sugar. Mucin includes secretory mucin produced by epithelial cell and the like and membrane-bound mucin that has a hydrophobic transmembrane site and exists while being bound to the cell membrane. The core proteins of mucin are collectively called MUC, and it is known that there are at least 20 types of genes encoding core proteins. One of them, mucin subtype 5AC (MUC5AC), belongs to secretory mucin.

MUC5AC is expressed in the stomach and trachea in normal tissues, and overexpression in pancreatic cancer has been reported. Overexpression has also been reported in thyroid cancer, liver cancer, colorectal cancer, gastric cancer, urothelial cancer, breast cancer, cervical cancer, ovarian cancer, endometrial cancer, and bile duct cancer. As antibodies to MUC5AC, a mouse antibody (Non-Patent Literature 1) prepared using, as an antigen, a pancreatic cancer mucin fraction purified from xenograft of human pancreatic cancer cell line SW1990, and chimeric antibodies (Patent Literatures 1, 2, Non-Patent Literatures 2, 3) and humanized antibody (Patent Literatures 3, 4) produced based thereon have been reported. In addition, radionuclide-labeled antibodies, such as humanized antibodies against MUC5AC labeled with $^{225}Ac$ or $^{89}Zr$, have been reported (Patent Literature 5).

In the formulation of radionuclide-labeled antibodies, Non Patent Literature 4 discloses a method of producing panitumumab in which $^{89}Zr$ is bound to deferoxamine, which is one type of ligand compound, and storing same in 0.9% physiological saline. In addition, Patent Literature 6 discloses the use of a surfactant alone or in combination with salts such as NaCl, KCl, $NaCl_2$ and $MgCl_2$ to suppress the radiolysis of peptides and proteins labeled with radionuclides.

Patent Literature 7, although not directed to the formulation of radionuclide-labeled antibodies, discloses that arginine can be useful as an initial stabilizer in liquid interleukin-2 (IL-2) pharmaceutical formulations to reduce aggregation and deamidation of IL-2 during long-term storage. In addition, Patent Literature 7 discloses that the inclusion of polysorbate 80 in the formulation is, while recognized as undesirable when used alone based on its effect on protein aggregation, advantageous during processing of liquid formulations containing this protein, and shows a stabilizing effect against severe protein damage associated with freeze-thawing and mechanical shear.

[Citation List]

[Patent Literature]

[Patent Literature 1]
JP H7-203974 A
[Patent Literature 2]
JP H11-5749 A
[Patent Literature 3]
WO 2013/157102
[Patent Literature 4]
WO 2013/157105
[Patent Literature 5]
WO 2021/075544
[Patent Literature 6]
JP H9-510454 A
[Patent Literature 7]
JP 2003-510368 A

[Non Patent Literature]

[Non Patent Literature 1]
Japanese Journal of Cancer Research, 87, 977-984, 1996
[Non Patent Literature 2]
Nippon Rinsho, Vol. 64, Extra Issue 1, 2006, 274-278
[Non Patent Literature 3]
Japanese Journal of Cancer Research, 90, 1179-1186, 1999
[Non Patent Literature 4]
J Labelled Comp Radiopharm. 57, 25-35, 2014

[Summary of Invention]

The present inventors have studied the formulation of an antibody that specifically binds to mucin subtype 5AC and is labeled with a radionuclide (hereinafter also to be referred to as "radioactive anti-MUC5AC specific antibody"), and found that the radioactive anti-MUC5AC specific antibody can be stabilized by using a specific amino acid. However, the present inventors have found a new problem of adsorption of radioactivity to storage containers. It is considered that the adsorption of radioactivity to storage containers is caused by one or both of the antibody and the radionuclide, which are constituent elements of the radioactive anti-MUC5AC specific antibody. When caused by the antibody, it is considered that the radioactive anti-MUC5AC specific antibody may be adsorbed to a storage container as a monomer, or that the radioactive anti-MUC5AC specific antibodies may aggregate with each other, or that the anti-MUC5AC specific antibody, which is a foreign substance contaminated in during production, and the radioactive anti-MUC5AC specific antibody, which is the active ingredient, may aggregate and adsorb to the storage container. Here, the anti-MUC5AC specific antibody as a foreign substance may be an aggregate of anti-MUC5AC specific antibodies, a fragmented anti-MUC5AC specific antibody, or an isomer with an altered glycan structure. Different from general antibody drugs that contain intact antibodies or antibody-drug conjugates in which non-radioactive drugs are attached to antibodies as active ingredients, since the amount of a radioactive anti-MUC5AC specific antibody as a substance contained in a preparation is very small, the adsorption of radioactivity to a storage container may affect the efficacy of the preparation.

In the formulation of radionuclide-labeled antibodies described in Non Patent Literature 4 and Patent Literature 6, the problem of radioactivity adsorption to storage containers is not recognized.

The present invention relates to the formulation of a pharmaceutical composition containing a radioactive anti-MUC5AC specific antibody as an active ingredient, and aims to provide a liquid pharmaceutical composition with reduced radioactivity adsorption to a storage container.

The inventors have conducted intensive studies in view of the above-mentioned problems and found that a liquid pharmaceutical composition prepared by a formula containing L-arginine, L-histidine, and a surfactant in the presence of a specific concentration of an anti-MUC5AC specific antibody reduces radioactive adsorption to a storage container, which resulted in the completion of the present invention.

One embodiment of the present invention is a liquid pharmaceutical composition containing a conjugate of a radionuclide and an antibody as an active ingredient, in which the antibody is an antibody that specifically binds to mucin subtype 5AC, the concentration of the antibody in the composition is 0.1 mg/mL or more and 5 mg/mL or less, and the composition also contains a stabilizer, a buffering agent, and a surfactant.

The concentration of the antibody in the composition in this embodiment is the total concentration of the antibody conjugated with the radionuclide and the antibody not conjugated with the radionuclide.

According to the present invention, since adsorption of radioactivity of a radioactive liquid pharmaceutical composition to a storage container is suppressed, a preparation of stable quality can be provided.

[Brief Description of Drawings]

[Fig. 1]
A schematic diagram for explaining the antibody concentration in the liquid pharmaceutical composition of the present invention.
[Fig. 2]
A diagram showing the results of the study on the effect of reducing adsorption of radioactive anti-MUC5AC specific antibody to a storage container, by the addition of a surfactant (polysorbate 80). The vertical axis indicates the degree of antibody adsorption to the storage container and the horizontal axis indicates the number of days after production of the pharmaceutical compositions.
[Fig. 3]
A diagram showing the results of flow imaging after transportation of preparations by road under refrigerated storage.

[Description of Embodiments]

Unless otherwise specified, the terms used in the present specification can be used with the meanings generally used in the pertinent technique field.

(1) Pharmaceutical composition

The present invention provides a liquid pharmaceutical composition (hereinafter also to be referred to as the liquid pharmaceutical composition of the present invention) containing, as an active ingredient, a conjugate of a radionuclide and an antibody (hereinafter also to be referred to as the conjugate of the present invention).

As used herein, the antibody to be contained in the liquid pharmaceutical composition and the conjugate of the present invention is an antibody that specifically binds to mucin subtype 5AC (hereinafter also to be referred to as the anti-MUC5AC specific antibody of the present invention). In the present specification, when the antibody is radiolabeled, it is indicated as "radioactive" for distinction.

The liquid pharmaceutical composition of the present invention is characterized in that it contains, in addition to the conjugate of the present invention as an active ingredient, L-arginine and L-histidine, and further a surfactant.

In the conjugate of radionuclide and antibody of the present invention, the antibody and radionuclide may be directly connected. In addition, in the conjugate of antibody and radionuclide of the present invention, antibody and radionuclide may be connected via a linker. In this case, the radionuclide may be a radioactive metal nuclide, and the radioactive metal nuclide may form a chelate (complex) with the chelating agent, and the chelating agent may be connected to the antibody via or not via a linker. The connection here may preferably be connection by a covalent bond.

(1-1) Radionuclide

The radionuclide contained in the conjugate of the present invention may be a radionuclide that emits $\alpha$ particles, positron, $\beta$-ray, or $\gamma$-ray. Examples of the radionuclide that emits $\alpha$ particles include Bi-212, Bi-213, At-211, Ac-225, Th-227. Examples of the radionuclide that emits positron include F-18, Cu-64, Ga-68, Y-86, Zr-89. Examples of the radionuclide that emits $\beta$-ray include Cu-64, Y-90, I-131 and Lu-177. Examples of the radionuclide that emits $\gamma$-ray include I-123, Tc-99m, and In-111. The radionuclide contained in the conjugate of the present invention, and thus in the liquid pharmaceutical composition of the present invention, is more preferably Zr-89, Y-90, Lu-177 or Ac-225.

These radionuclides can be produced by a predetermined nuclear reaction, or can be obtained as commercial products available from Eckert & Ziegler, Thermo Fisher Scientific, Institute of Isotopes, POLATOM, Oak Ridge National Laboratory, Rosatom, and the like. The radionuclides thus produced or obtained can be used to form a conjugate with an antibody by subjecting them to a chemical treatment such as chelating to make them into a chemical form suitable for binding to an antibody.

(1-2) Anti-MUC5AC specific antibody

The anti-MUC5AC specific antibody to be contained in the conjugate of the present invention is an immunoglobulin that specifically binds to mucin subtype 5AC. The anti-MUC5AC specific antibody to be used in the present invention may be a polyclonal antibody or a monoclonal antibody, preferably a monoclonal antibody. The origin of the antibody is not particularly limited, and examples include antibodies of non-human animals, non-human mammals, and humans, preferably antibodies of human, rat, mouse, and rabbit. When the antibody is derived from species other than human, it is preferably chimerized or humanized using well-known techniques. The antibody to be used in the present invention may be a chimeric antibody, a humanized antibody, or a human antibody. The antibody to be used in the present invention is, for example, IgG, for example, IgG1, IgG2 **(e.g.,** IgG2a or IgG2b), IgG3, or IgG4.

Preferably, the antibody is a humanized antibody having the ability to specifically bind to MUC5AC, has stable physical properties, and is superior in tumor accumulation. The antibody may be used as an antigen-binding fragment thereof, and such embodiment is also encompassed in the present invention. Specifically, it contains a specific heavy chain variable region and a specific light chain variable region described below and, when desired, contains an appropriate heavy chain constant region and an appropriate light chain constant region. In the present specification, the "antigen-binding fragment" means an antibody fragment consisting of a part of the humanized antibody used in the present invention, and having the binding ability to MUC5AC. The number of amino acids contained in the polypeptide constituting the antigen-binding fragment is not particularly limited as long as it has the binding ability to MUC5AC.

Amino acid sequences preferred as the heavy chain variable region of the humanized antibody used in the present invention are shown below. The heavy chain variable region 1 (H01), heavy chain variable region 2 (H02), heavy chain variable region 3 (H03), and heavy chain variable region 4 (H04) respectively correspond to SEQ ID NOs: 1 to 4 in the Sequence Listing attached to the present specification. The underlined parts are CDR.

[Chem. 1]

[heavy chain variable region 1 (H01)]

E V Q L L E S G G G L V Q P G G S L R L S C A A S G F T F S N Y G M S W V R
Q A P G K G L E W V S T I S N S G R Y T Y F P D S V K G R F T I S R D N S K
N T L Y L Q M N S L R A E D T A L Y Y C T R H L D Y A N Y D A M D Y W G Q G
T L V T V S S

[heavy chain variable region 2 (H02)]

L V Q L V E S G G G V V R P G G S L R L S C A A S G F T F S N Y G M S W I R
Q A P G K G L E W V S T I S N S G R Y T Y F P D S V K G R F T I S R D N A K
N S L Y L Q M N S L R A E D T A L Y Y C T R H L D Y A N Y D A M D Y W G Q G
T L V T V S S

[heavy chain variable region 3 (H03)]

L V Q L V E S G G G V V Q P G R S L R L S C A A S G F T F S N Y G M S W V R
Q A P G K G L E W V A T I S N S G R Y T Y F P D S V K G R F T I S R D N S K
N T L Y L Q M N S L R A E D T A V Y Y C T R H L D Y A N Y D A M D Y W G Q G
T L V T V S S

[heavy chain variable region 4 (H04)]

E V Q L L E S G G G L V Q P G G S L R L S C A V S G F T F S N Y G M S W V R
Q A P G K G L E W V S T I S N S G R Y T Y F P D S V K G R F T I S R D N S R
N T L Y L Q M N T L R A E D T A V Y Y C T R H L D Y A N Y D A M D Y W G Q G
T P V T V S S

Amino acid sequences preferred as the light chain variable region of the humanized antibody used in the present invention are shown below. The light chain variable region 1 (L01), light chain variable region 2 (L02), light chain variable region 3 (L03), and light chain variable region 4 (L04) respectively correspond to SEQ ID NOs: 5 to 8 in the Sequence Listing attached to the present specification. The underlined parts are CDR.

[Chem. 2]

[light chain variable region 1 (L01)]

D I V M T Q S P S S L S A S V G D R V T I T C R A S K S V T T S D F S Y M H
W Y Q Q K P G K A P K L L I Y L A S N L E S G V P S R F S G S G S G T D F T
L T I S S L Q P E D F A T Y Y C Q H S R E F P W T F G G G T K V E I K

[light chain variable region 2 (L02)]

D V V M T Q S P S T L S A S V G D R V T I T C R A S K S V T T S D F S Y M H
W Y Q Q K P G Q A P K L L I Y L A S N L E S G V P S R F S G S G S G T D F T
L T I S S L Q P E D F A T Y Y C Q H S R E F P W T F G Q G T K L E I K

[light chain variable region 3 (L03)]

D I Q M T Q S P S S L S A S V G D R V T I T C R A S K S V T T S D F S Y M H
W Y Q Q K P G K S P K L L I Y L A S N L E S G V P S R F S G S G S G T D F S
L T I S S L Q P E D F A T Y Y C Q H S R E F P W T F G G G T K V E I K

[light chain variable region 4 (L04)]

D I V M T Q S P D S L A V S L G E R A T I N C K A S K S V T T S D F S Y L H
W Y Q Q K P G Q P P K L L I Y L A S N L E S G V P D R F S G S G S G T D F T
L T I S S L Q A E D V A V Y Y C Q H S R E F P W T F G G G T K L E I K

In other words, the heavy chain variable region of the MUC5AC specific antibody preferred in the present invention consists of the amino acid sequence shown in any one of SEQ ID NO: 1 to SEQ ID NO: 4, and the light chain variable region consists of the amino acid sequence shown in any one of SEQ ID NO: 5 to SEQ ID NO: 8. That is, the MUC5AC specific antibody used in the present invention consists of a combination of any one of the above-mentioned four heavy chain variable regions (H01 - H04) and any one of the four light chain variable regions (L01 - L04).

A preferred MUC5AC specific antibody in the present invention has heavy chain variable region H01, H03, or H04, and any one of L01 - L04 as the light chain variable region.

The most preferred MUC5AC specific antibody in the present invention has heavy chain variable region H01 and light chain variable region L03.

The heavy chain variable region of the MUC5AC specific antibody in the present invention is not limited to those defined by the amino acid sequence shown in SEQ ID NO: 1 to SEQ ID NO: 4 and also includes variants maintaining functions. That is, a mutated heavy chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 1 to SEQ ID NO: 4 is also used as the heavy chain variable region of the MUC5AC specific antibody to be used in the present invention as long as it can bind to MUC5AC when combined with the light chain variable region in the present invention.

In the present specification, the identity of the amino acid sequence refers to the identity of the amino acid sequences between the two proteins of interest, and is shown by the percentage (%) of amino acid residues that match in the optimal alignment of the amino acid sequences prepared using mathematical algorithms known in the pertinent technical field. The identity of an amino acid sequence can be determined by visual inspection and mathematical calculation, and can be calculated using a homology search program (e.g., BLAST, FASTA) or sequence alignment program (e.g., ClustalW) known to those skilled in the art, or genetic information processing software (e.g., GENETYX [registered trademark]), and the like. To be specific, the identity of the amino acid sequence in the present specification can be determined using systematic analysis program ClustalW
(http://clustalw.ddbj.nig.ac.jp/index.php?lang=ja) published on the website of DDBJ (DNA DataBank of Japan) by the initial setting conditions (Version2.1, Alignment type: slow, DNA Weight Matrix: Gonnet, GAP OPEN: 10, GAP EXTENSION: 0.1).

In addition, as the heavy chain variable region of the MUC5AC specific antibody to be used in the present invention, a mutated heavy chain variable region consisting of the amino acid sequence shown in SEQ ID NO: 1 to SEQ ID NO: 4, wherein not more than 10, preferably not more than 8, further preferably not more than 5, most preferably not more than 3 (3, 2 or 1), amino acids are deleted, substituted, or added, is also used as the heavy chain variable region of the MUC5AC specific antibody to be used in the present invention as long as it can bind to MUC5AC when combined with the light chain variable region in the present invention.

The light chain variable region of the MUC5AC specific antibody to be used in the present invention is not limited to the amino acid sequence shown in SEQ ID NO: 5 to SEQ ID NO: 8 and also includes variants maintaining functions. That is, a mutated light chain variable region consisting of an amino acid sequence having not less than 90%, preferably not less than 95%, not less than 96%, or not less than 97%, further preferably not less than 98%, most preferably not less than 99%, sequence identity with the amino acid sequence shown in SEQ ID NO: 5 to SEQ ID NO: 8 is also used as the light chain variable region of the MUC5AC specific antibody to be used in the present invention as long as it can bind to MUC5AC when combined with the heavy chain variable region in the present invention.

In addition, as the light chain variable region of the MUC5AC specific antibody to be used in the present invention, a mutated light chain variable region consisting of the amino acid sequence shown in SEQ ID NO: 5 to SEQ ID NO: 8, wherein not more than 10, preferably not more than 8, further preferably not more than 5, most preferably not more than 3 (3, 2 or 1), amino acids are deleted, substituted, or added, is also encompassed in the light chain variable region of the MUC5AC specific antibody to be used in the present invention as long as it can bind to MUC5AC when combined with the heavy chain variable region in the present invention.

The MUC5AC specific antibody to be used in the present invention can be produced by a method generally performed in the art or a method analogous thereto. Specifically, the steps described in WO 2021/075544 and WO 2021/075545 can be performed.

(1-3) Chelating agent

In the present invention, the chelating agent is not particularly limited as long as it has a site in the structure thereof where radionuclide is coordinated. Preferably, it has a chelating moiety, which is a site to which a radionuclide is

coordinated, and a substituent that enables conjugating with an antibody. Examples of the chelating moiety include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α",α'"-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-Carboxyethyl)-1,4,7,10-tetraazacyclododecane-1,4,7,10-tetraacetic acid), DOTA-GA-NHS, DOTP (((1,4,7,10-Tetraazacyclododecane-1,4,7,10-tetrayl)tetrakis(methylene))tetraphosphonic acid), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane($L^{py}$), p-SCN-Bn-DOTA (S-2-(4-Isothiocyanatobenzyl)-1,4,7,10-tetraazacyclododecane tetraacetic acid), MeO-DOTA-NCS (1-[(2-methoxy-5-isothiocyanatophenyl)-carboxymethyl]-4,7,10-triscarboxy 5 methyl-1,4,7,10-tetraazacyclododecane), EuK-106, DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), DTPA-BMA (5,8-Bis(carboxymethyl)-11-[2-(methylamino)-2-oxoethyl]-3-oxo-2,5,8,11-tetraazatridecan-13-oic acid), EDTA (2,2',2",2'"-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid), TETPA (1,4,8,11-tetraazacyclotetradecane-1,4,8,11-tetrapropionic acid), TETA (1,4,8,11-Tetraazacyclotetradecane-N,N',N",N'"-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N'"-tetra(1,2-dihydro-1-hydroxy-2-oxopyridine-6-carbonyl)-1,5,10,14-tetraazatetradecane), PEPA (1,4,7,10,13-pentaazacyclopentadecane-N,N',N",N'",N""-penta-acetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridine-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), porphyrin, DO3A (1,4,7,10-Tetraazacyclododecane-1,4,7-triacetic acid trisodium salt), and DO3A-NHS (1,4,7,10-Tetraazacyclododecane-1,4,7,10-tetraacetic acid mono-N-hydroxysuccinimide ester). It is preferable to have a structure derived from a compound represented by the following formula (A).

[Chem. 3]

(A)

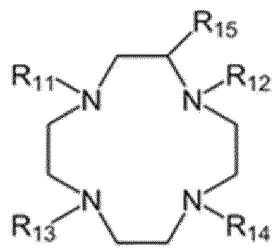

In the formula (A), $R_{11}$, $R_{13}$ and $R_{14}$ are each independently a group consisting of $-(CH_2)_pCOOH$, $-(CH_2)_pC_5H_4N$, $-(CH_2)_pPO_3H_2$, $-(CH_2)_pCONH_2$ or $-(CHCOOH)(CH_2)_pCOOH$, one of $R_{12}$ and $R_{15}$ is a hydrogen atom, a carboxyl group, or a carboxyalkyl group having 2 or 3 carbon atoms, the other is a substituent for conjugating with the aforementioned antibody, p is an integer of not less than 0 and not more than 3, $R_{15}$ is a hydrogen atom when $R_{12}$ is a substituent for conjugating with the aforementioned antibody, and $R_{15}$ is a substituent for conjugating with the aforementioned antibody when $R_{12}$ is not a substituent for conjugating with the aforementioned antibody.

Examples of the specific structure represented by the formula (A) include structures derived from the compounds represented by the following formulas (A-1) to (A-12).

[Chem. 4]

(A-1)

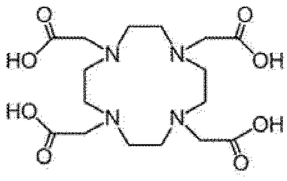

DOTA

(A-2)

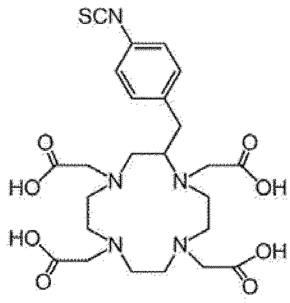

p-SCN-Bn-DOTA

(A-3)

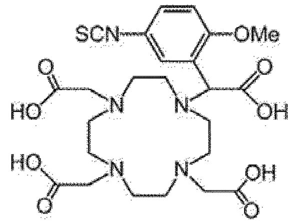

MeO-DOTA-NCS

(A-4)

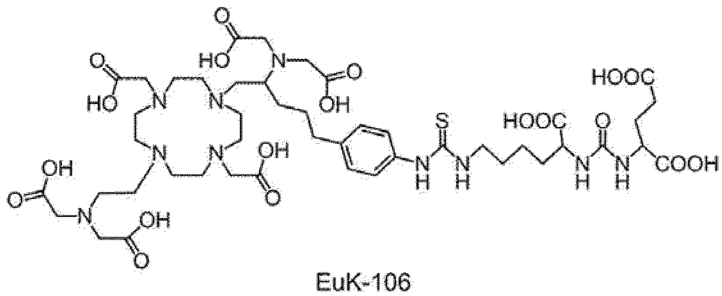

EuK-106

[Chem. 5]

(A-5)

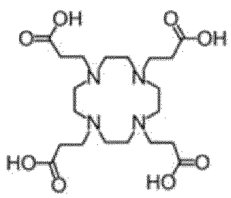

DOTPA

(A-6)

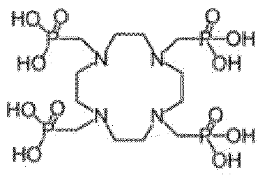

DOTMP

(A-7)

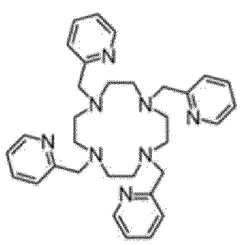

$L^{py}$

(A-8)

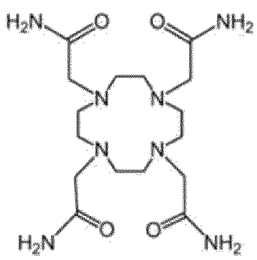

DOTAM

(A-9)

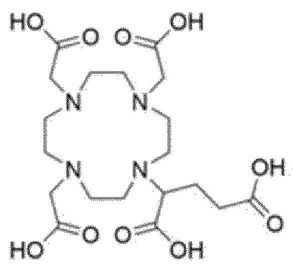

DOTA-GA

[Chem. 6]

(A-10)

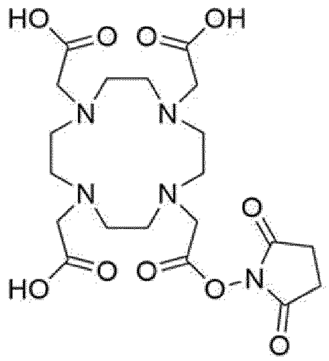

DO3A-NHS

(A-11)

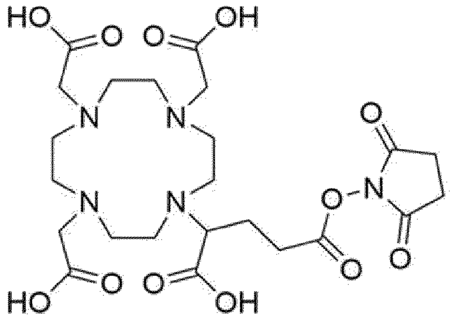

DOTA-GA-NHS

(A-12)

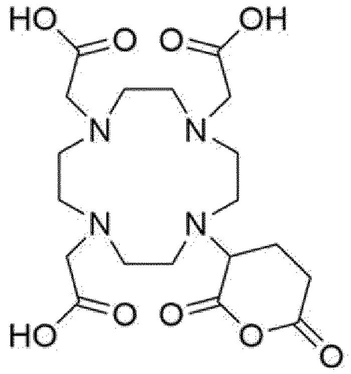

DOTA-GA-anhydride

The linkage site between the chelating moiety and the substituent that enables formation of a conjugate with the antibody is preferably an amide bond or a thiourea bond, more preferably an amide bond from the aspect of stability.

The amide bond can be formed, for example, by the reaction of an N-hydroxysuccinimide ester (NHS) group of the above-mentioned formula (A-10) or (A-11), or a 2,6-dioxo tetrahydro-2H-pyranyl group of the above-mentioned (A-12) with primary amine. The thiourea bond can be formed by the reaction of an isothiocyanate group of the compound of the above-mentioned formula (A-2), (A-3) with primary amine or maleimide group.

When the conjugate of the present invention is a conjugate of a chelating agent with radionuclide chelated and an antibody, the chelating agent may be provided at least not less than one molecule, preferably not less than 1 molecule and not more than 8 molecules, per one molecule of the antibody. To maintain the activity of the antibody itself (antigen recognition action, neutralizing action, complement activating action and/or opsonin action), a chelating agent is preferably introduced site-specifically into the Fc region (constant region) of the antibody. In the present invention, the chelating agent is preferably provided at one or two molecules per one molecule of the antibody. The chelating agent is more preferably provided at one molecule per one molecule of the antibody.

In the conjugate of the present invention, the chelating agent may be connected to the antibody via a linker. Examples of the linker include substituted or unsubstituted alkyl group, substituted or unsubstituted heteroalkyl group, polyethylene glycol (PEG) group, peptides, sugar chain, disulfide group, combination of these and the like.

Preferably, the chelating agent modifies the antibody site-specifically, more preferably in the Fc region, via a linker. In this case, the linker contains a peptide consisting of not less than 13 and not more than 17 amino acid residues represented by the following formula (i) (hereinafter, to be also referred to as "antibody-modification peptide"), and one formed by a cross-linking reaction between the antibody-modification peptide modified with a crosslinking agent and the antibody can be used. In the explanation of the formula (i), the left side of the amino acid sequence on the page indicates the N-terminal side, and the right side of the amino acid sequence on the page indicates the C-terminal side. When the chelating agent is connected to the antibody via the antibody-modification peptide as a linker, the position where the chelating agent and the antibody-modification peptide are linked is not particularly limited. For example, it can be directly or indirectly linked at the N-terminal or C-terminal of the antibody-modification peptide, preferably at the N-terminal. In addition, the C-terminal of the antibody-modification peptide may be modified by, for example, amidation or the like to improve its stability and the like.

(Xa) -Xaa$_1$-(Xb) -Xaa$_2$-(Xc) -Xaa$_3$-(Xd) (i)

In the formula (i), Xa, Xb, Xc and Xd are each continuous X in the number of a, continuous X in the number of b, continuous X in the number of c, and continuous X in the number of d, respectively,

X is an amino acid residue having neither a thiol group nor a haloacetyl group in the side chain,

a, b, c and d are each independently an integer of not less than one and not more than 5, and satisfy $a+b+c+d \leq 14$,

Xaa$_1$ and Xaa$_3$ are each independently an amino acid residue derived from an amino acid having a thiol group in the side chain, or

one is an amino acid residue derived from an amino acid having a thiol group in the side chain and the other is an amino acid residue derived from an amino acid having a haloacetyl group in the side chain, and Xaa$_1$ and Xaa$_3$ are linked, and

$Xaa_2$ is a lysine residue, arginine residue, cysteine residue, aspartic acid residue, glutamic acid residue, 2-aminosuberic acid, or diamino propionic acid, and modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

In the formula (i), a, b, c and d are not particularly limited as long as they are numbers within the aforementioned range. From the aspect of the stability of binding between the peptide and antibody, $a+b+c+d \leq 14$ is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

$Xaa_1$ and $Xaa_3$ are amino acid residues derived from an amino acid having a thiol group in the side chain, and $Xaa_1$ and $Xaa_3$ may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the wavy line indicates the binding part with the sulfide group.

[Chem. 7]

(4) 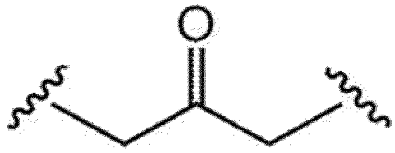

Instead of the aforementioned combination of $Xaa_1$ and $Xaa_3$, one of $Xaa_1$ and $Xaa_3$ may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Among these, the amino acid sequence of the antibody-modification peptide preferably has any one of the following sequences (1) to (14), more preferably the following sequence (1), (2), (13) or (14). In the following amino acid sequences (1) to (14), ($Xaa_2$) is a lysine residue, a cysteine residue, an aspartic acid residue, a glutamic acid residue, a 2-aminosuberic acid, or a diamino propionic acid, and ($Xaa_1$) and ($Xaa_3$) are each a homocysteine residue. In the following amino acid sequences (1) to (14), the amino acids other than ($Xaa_1$), ($Xaa_2$) and ($Xaa_3$) are indicated by one-letter abbreviations.

(1) DCAYH($Xaa_2$)GELVWCT (SEQ ID NO: 9)
(2) GPDCAYH($Xaa_2$)GELVWCTFH (SEQ ID NO: 10)
(3) RCAYH($Xaa_2$)GELVWCS (SEQ ID NO: 11)
(4) GPRCAYH($Xaa_2$)GELVWCSFH (SEQ ID NO: 12)
(5) SPDCAYH($Xaa_2$)GELVWCTFH (SEQ ID NO: 13)
(6) GDDCAYH($Xaa_2$)GELVWCTFH (SEQ ID NO: 14)
(7) GPSCAYH($Xaa_2$)GELVWCTFH (SEQ ID NO: 15)
(8) GPDCAYH($Xaa_2$)GELVWCSFH (SEQ ID NO: 16)
(9) GPDCAYH($Xaa_2$)GELVWCTHH (SEQ ID NO: 17)
(10) GPDCAYH($Xaa_2$)GELVWCTFY (SEQ ID NO: 18)
(11) SPDCAYH($Xaa_2$)GELVWCTFY (SEQ ID NO: 19)
(12) SDDCAYH($Xaa_2$)GELVWCTFY (SEQ ID NO: 20)
(13) RGNCAYH($Xaa_2$)GQLVWCTYH (SEQ ID NO: 21)
(14) G($Xaa_1$)DCAYH($Xaa_2$)GELVWCT($Xaa_3$)H (SEQ ID NO: 22)

(1-4) Production method of conjugate

The production method of the conjugate of the present invention is described.

The anti-MUC5AC specific antibody of the present invention can be maintained in a buffer.

The conjugate of the present invention can be prepared, for example, by introducing radioactive iodine ($^{123}I$, $^{131}I$) as a radionuclide into the tyrosine residue of the anti-MUC5AC specific antibody. Alternatively, the conjugate may be prepared by introducing a substituent to which a radioactive halogen nuclide stably binds, into the anti-MUC5AC specific antibody of the present invention and reacting the resulting antibody with a radioactive halogen ion.

When the conjugate of the present invention is a conjugate of a chelating agent with radionuclide chelated and an antibody, the conjugate of the present invention can be produced by two steps of a conjugation step of conjugating a chelating agent and an antibody, and a complex formation step of forming a complex of a radionuclide and a chelating agent (production step of chelating agent with radionuclide chelated). The conjugation step may be performed before the complex formation step or after the complex formation step.

In the conjugation step, various methods for chemical modification of antibody are used. Specifically, the methods (a) - (f) can be mentioned:

(a) amine coupling method (a method for modifying the amino group of a lysine residue of an antibody by using a chelating agent or chelate having a carboxyl group activated by an **N-**hydroxysuccimidyl (NHS) group)
(b) method for modifying a sulfhydryl (SH) group generated by partially reducing a disulfide bond (SS bond) between polypeptide chains at the hinge site of an antibody with a chelating agent or linker having a maleimide group reactive with the SH group
(c) method for modifying cysteine newly introduced into an antibody by an amino acid mutation by genetic engineering with a chelating agent or linker having a maleimide group
(d) method for modifying an azide group of lysine azide newly introduced into an antibody by an amino acid mutation by genetic engineering with a chelating agent or linker having alkyne (e.g., Dibenzylcyclooctyne: DBCO) by using a click reaction
(e) method for modifying glutamine introduced into a specific position of an antibody with a chelating agent or linker having a side chain of lysine by using transglutaminase
(f) method for site-specifically modifying the Fc region of an antibody with a chelating agent or linker having the antibody-modification peptide shown in the aforementioned (i)

In the complex formation step, the chelating agent is chelated with a radionuclide (complex formation). The radionuclide used here is preferably used in a manner permitting ionization, more preferably in the form of an ion, from the viewpoint of increasing the complex formation efficiency. In the complex formation step, the order of addition of the radionuclide to the chelating agent does not matter as long as a complex of a radionuclide can be formed. For example, a solution in which radionuclide ions are dissolved in a solvent mainly composed of water can be used as a radionuclide.

After complex formation, the obtained complex may be purified using a filtration filter, a membrane filter, a column filled with various fillers, chromatography or the like.

In the production method of the chelate conjugate of the present invention, a conjugation step is preferably performed after the complex formation step.

In a more preferred embodiment, in complex formation step (A), a complex is formed between a radionuclide and a chelating agent having a first atomic group capable of click reaction as a substituent for enabling conjugation with the antibody. Then, in conjugation step (B), using an antibody-modification peptide shown by the aforementioned (i) and an antibody-modification linker having a second atomic group capable of click reaction, a click reaction is performed between the peptide-modified antibody in which Fc region is site-specifically modified and the chelating agent with a formed complex which is obtained in step (A) to obtain the chelate conjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker is preferably alkyne and the antibody-modification linker is preferably azide, or the chelate linker is preferably 1,2,4,5-tetrazine and the antibody-modification linker is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or a combination of an atomic group containing 1,2,4,5-tetrazine as the first atomic group (the formula (1b)) and an atomic group containing trans-cyclooctene (TCO) as alkene of the second atomic group (the formula (2b)). Preferred is the

combination of the formula (1a) and the formula (2a).

[Chem. 8]

(1a) 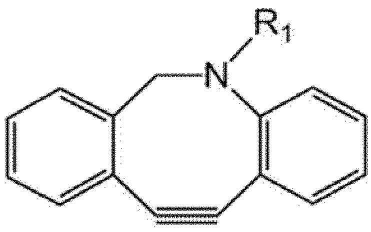

Dibenzylcyclooctyne

(2a) 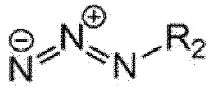

Azide

wherein $R_1$ is a linkage site with a chelating agent, and $R_2$ is a linkage site with an antibody-modification peptide in the antibody.

[Chem. 9]

(1b) 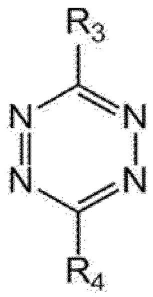

1,2,4,5-tetrazine

(2b) 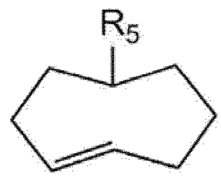

*trans*-cyclooctene

wherein one of $R_3$ and $R_4$ is a linkage site with a chelating agent or an antibody-modification peptide in the antibody, and the other is a hydrogen atom, a methyl group, a phenyl group or a pyridyl group, and $R_5$ is a linkage site with any one chelating agent or an antibody-modification peptide in the antibody depending on $R_3$ or $R_4$.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-NHS ester, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In step (A), more preferably, a chelating agent having a structure represented by the following formula (ii) is used.

A-B-C (ii)

In the formula (ii), A is a chelating moiety represented by the following formula (iia).

[Chem. 10]

(iia) 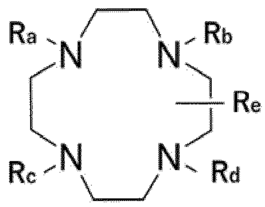

In the formula (iia), Ra, Rb, and Rc are each independently a group consisting of $-(CH_2)_pCOOH$, $-(CH_2)_pC_5H_4N$, $-(CH_2)_pPO_3H_2$, $-(CH_2)_pCONH_2$, or $-(CHCOOH)(CH_2)_pCOOH$, p is an integer of not less than 0 and not more than 3, either Rd or Re is a binding site (* in the following formula (iib)) to B, and the other is a hydrogen atom or a group consisting of $-(CH_2)_pCOOH$, $-(CH_2)_pC_5H_4N$, $-(CH_2)pPO_3H_2$, $-(CH_2)_pCONH_2$, or - $(CHCOOH)(CH_2)_pCOOH$, and p is an integer of not less than 0 and not more than 3.

In the formula (ii), B is represented by the following formula (iib):

[Chem. 11]

(iib) 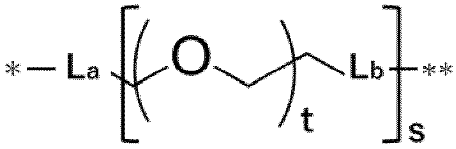

In the formula (iib), La and Lb are each independently a bond linker containing at least an amide bond or thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

[Chem. 12]

(iic) 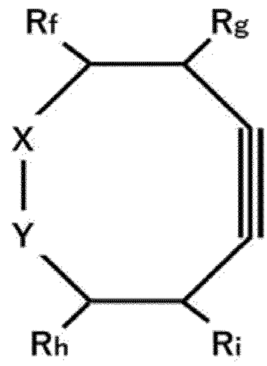

(iid) 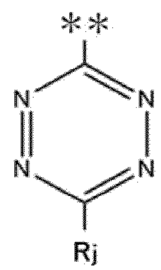

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (iia) wherein Ra to Rd are $-(CH_2)_pCOOH$, p is 1, Re is a binding site with B; or DO3A derivative or DOTA-GA derivative wherein Ra to Rc are $-(CH_2)_pCOOH$, p is **1,** Rd is a binding site with **B,** and Re is a hydrogen atom is more preferred.

In the formula (ii), a DOTA-PEGt-DBCO derivative wherein A is the above-mentioned DOTA derivative, in **B,** La is a bond linker containing a thiourea bond and having not less than 1 and not more than 50 carbon atoms, s is 0 or **1,** when s is **1, t** is an integer of not less than 0 and not more than 30, Lb is a bond linker containing an amide bond or thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh

and Ri are jointed to form a benzene ring, and ** is a binding site with B; or a DOTA-PEGt-Tz derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or thiourea bond and having not less than 1 and not more than 50 carbon atoms, and C is a tetrazine derivative represented by the formula (iid) is further preferred.

In the formula (ii), a DO3A-PEGt-DBCO derivative wherein A is the above-mentioned DO3A derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the formula (ii), a DOTA-GA-PEGt-DBCO derivative wherein A is the above-mentioned DOTA-GA derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further preferred.

In the molar ratio of the chelating agent and radionuclide, as chelating moiety/radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

In the reaction time, provided that the reaction temperature is as described above, lower limit is preferably not less than 5 minutes, more preferably not less than 10 minutes, further preferably not less than 20 minutes, further more preferably not less than 30 minutes, particularly preferably not less than 45 minutes, and the upper limit is preferably not more than 180 minutes, more preferably not more than 150 minutes, further preferably not more than 120 minutes, further more preferably not more than 90 minutes, and especially not more than 60 minutes, for example, and a range of not less than 10 minutes and not more than 150 minutes is preferred, and not less than 10 minutes and not more than 60 minutes is more preferred.

The antibody to be used in step (B) is a peptide-modified antibody in which Fc region (constant region) of humanized antibody as described in detail in the above-mentioned "(1-2) Antibody" is site-specifically modified using the antibody-modification peptide shown in the aforementioned (i), and an antibody-modification linker having the second atomic group capable of click reaction.

The antibody-modification peptide can be produced using a combination of amino acids regardless of natural amino acids and unnatural amino acids, by subjecting to peptide synthesis methods such as liquid phase synthesis

process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker may be one in which an antibody-modification peptide and a linker represented by the following formula (S1) are bonded.

$$*\text{-}((L_1)_m\text{-}Z)_k\text{-}L_2\text{-}AG_2 \qquad (S1)$$

wherein * is a binding site with the N-terminal or C-terminal of peptide,

$L_1$ is a linker moiety of polyethylene glycol (PEG),
m is an integer of not less than 1 and not more than 50,
Z is a second linker moiety that binds $(L_1)_m$ and $L_2$,
k is 0 or 1,
$L_2$ is the second PEG linker moiety, and
$AG_2$ is a second atomic group.

In the aforementioned formula (S1), the structure of Z is not particularly limited as long as it is a linker structure that binds $(L_1)_m$ and $L_2$ to each other, and includes, for example, an amino acid sequence consisting of not less than 1 and not more than 5 amino acid residues. In this case, the amino acid sequence contained in Z preferably contains a cysteine residue, and is more preferably bonded to $L_2$ via a thioether group formed by the bond between the thiol group of the cysteine residue and a maleimide group.

In the present invention, the PEG linker moiety constituting $L_2$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

[Chem. 13]

(P2) 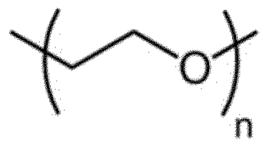

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker, an introduction method including obtaining an antibody-modification peptide having a desired amino acid sequence by the aforementioned method, dissolving the peptide in a solution containing a solubilizing agent and a reducing agent and where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the **N**-terminal or C-terminal of a peptide by using a commercially available click chemistry reagent containing TCO according to a conventional method, or an atomic group containing TCO can be introduced via the aforementioned linker structure.

The method for binding an antibody-modification peptide to an antibody to obtain a peptide-modified antibody can be performed, for example, using a crosslinking agent. A crosslinking agent is a chemical substance for linking an antibody-modification peptide and an antibody by a covalent bond. Examples thereof include a crosslinking agent

preferably containing two or more succinimidyl groups such as disuccinimidyl glutarate (DSG), disuccinimidyl suberate (DSS) and the like, a crosslinking agent consisting of a compound containing two or more imidic acid moieties such as dimethyl adipimidate and the like, or a salt thereof, a crosslinking agent consisting of a compound having a disulfide bond such as dimethyl 3,3'-dithiobispropionimidate, dithiobissuccinimidylpropionic acid, and the like, or a salt thereof, and the like. Using such crosslinking agent, a crosslinking reaction can be caused between an amino acid residue of Xaa2 in the antibody-modification peptide and an antibody. When, for example, the humanized antibody of the present invention is used as the antibody, the crosslinking reaction in the antibody occurs site-specifically between an amino acid residue of Xaa2 and a Lys246 or Lys248 residue according to the Eu numbering in the humanized antibody of the present invention. These Lys residues are present in the Fc region of the humanized antibody of the present invention.

The method for binding the antibody-modification peptide to the antibody can be performed, for example, by dispersing the aforementioned antibody-modification peptide, an antibody, a crosslinking agent, and a catalyst as necessary in an appropriate buffer at not less than 10°C and not more than 30°C. The reaction time may be about not less than 10 min to 2 hr or less. The molar ratio at the time of reaction of the peptide and the antibody is preferably not less than 1/5, more preferably not less than 1/3, further preferably not less than 1/1.5 as the lower limit of the antibody/peptide, and the upper limit is preferably not more than 20/1, more preferably not more than 10/1, further preferably not more than 5/1, further more preferably not more than 1/1, particularly preferably not more than 1/1.7. For example, the range of not less than 1/5 and not more than 20/1 is preferable, and not less than 1/1.5 and not more than 1/1.7 is more preferable.

The peptide-modified antibody obtained through the above steps is a mixture containing an antibody in which one molecule of antibody-modification peptide is bound to one molecule of an antibody (hereinafter to be referred to as "monovalent antibody") and an antibody in which two molecules of antibody-modification peptide are bound to one molecule of an antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is preferable to use a column packed with various fillers, and it is more preferable to use a column packed with a filler suitable for separation and purification of proteins such as antibody and the like.

The filler suitable for separation and purification of protein such as antibody and the like is not particularly limited as long as it is a filler in which an immunoglobulin-binding protein is immobilized on a carrier composed of a water-insoluble substrate, and which specifically binds to an antibody. Examples of the immunoglobulin-binding protein include protein A, protein G, protein L and the like. These immunoglobulin-binding proteins may be genetically engineered recombinants. Examples of the recombinant immunoglobulin-binding protein include genetically-engineered protein A, genetically-engineered protein G, and fused protein A domain and protein G domain. In the present invention, as a filler suitable for separation and purification of at least a monovalent antibody and a divalent antibody, protein A is more preferred, and genetically-engineered protein A is further more preferred. As used herein, protein A and protein G are protein molecules that can specifically bind to an antibody molecule IgG, and classified as protein A (Staphylococcus aureus) or protein G (streptococcus: Streptococcus genus) depending on the difference in the origin of the isolated microorganisms. The genetically-engineered protein A is a protein A in which at least one amino acid mutation has been introduced into an amino acid residue of any of the IgG binding domains (E, D, A, B and C domains) of protein A. In the present invention, genetically-engineered protein A in which the domain into which at least one amino acid mutation has been introduced is multimerized is preferable, genetically-engineered protein A in which A, B or C domain into which at least one amino acid mutation of protein A has been introduced is multimerized is more preferable, and genetically-engineered protein A multimerized to not less than a dimer and not more than a pentamer is further more preferable. The amino acid mutation may be derived from any mutation such as substitution, deletion, insertion and the like of the amino acid sequence or the base sequence encoding the amino acid in the transcriptional translation step of the gene. Examples thereof that are not particularly limited include the genetically-engineered protein A described in WO 2003/080655, WO 2011/118699 and the like.

Examples of the water-insoluble substrate on which immunoglobulin-binding proteins are immobilized include inorganic carriers such as glass beads, silica gel and the like, organic carriers such as synthetic polymers (e.g., crosslinked polyvinyl alcohol, crosslinked polyacrylate, crosslinked polyacrylamide, crosslinked polystyrene) and polysaccharides (e.g., crystalline cellulose, crosslinked cellulose, crosslinked agarose, crosslinked dextran), and organic-organic, organic-inorganic conjugate carriers and the like obtained from combinations of these, and the like.

The column filled with the aforementioned genetically-engineered protein A as a filler is commercially available as, for example, KanCap (registered trademark) series (KANEKA KanCapA prepacked column) of KANEKA CORPORATION, HiTrap (registered trademark) series (HiTrap Mabselect, HiTrap Mabselect SuRe, HiTrap Mabselect Xtra) of GE Healthcare, HiScreen series (HiScreen Mabselect SuRe) of GE Healthcare, TOYOPEARL (registered trademark) series

(TOYOPEARL AF-rProteinA-650F) of Tosoh Corporation, and the like.

The separation and purification of peptide-modified antibody used for click reaction in step (B) is explained below as an example.

A peptide-modified antibody is subjected to a click reaction in step (B) after an antibody modification step in which a modified antibody is obtained by site-specifically modifying an Fc region of an antibody by a linker provided with an antibody-modification peptide (antibody-modification linker), and an antibody purification step in which the modified antibody is purified using the aforementioned carrier with an immunoglobulin-binding protein immobilized thereon. In addition, the antibody purification step further includes a retention step of retaining the modified antibody retained on the carrier, a washing step of washing the modified antibody not retained on the carrier, and an elution step of eluting the modified antibody retained on the carrier in the retention step.

More specifically, in the antibody modification step, a modified antibody is obtained as a mixture containing an unmodified antibody not modified by an antibody-modification linker, a monovalent antibody, and a divalent antibody and, in the antibody purification step, the first antibody composition containing relatively large amounts of the unmodified antibody and the monovalent antibody and the second antibody composition containing a relatively large amount of the divalent antibody are respectively eluted utilizing the difference in the interaction of the unmodified antibody, monovalent antibody and divalent antibody with immunoglobulin-binding proteins. That is, in the retention step and washing step among the antibody purification steps, the second antibody composition containing a relatively large amount of peptide-modified antibody (divalent antibody) having a low degree of interaction with immunoglobulin-binding proteins is eluted and, in the elution step among the antibody purification steps, the first antibody composition containing a relatively large amount of peptide-modified antibody (unmodified antibody and monovalent antibody) having a high degree of interaction with immunoglobulin-binding proteins is eluted. As used herein, "containing a relatively large amount of unmodified antibody and monovalent antibody" means that the total amount of unmodified antibody and monovalent antibody contained in the first antibody composition is larger than that of the divalent antibody contained in the antibody composition, preferably that the total amount of the unmodified antibody and monovalent antibody is not less than 55%, not less than 63%, not less than 70%, not less than 80%, or not less than 90%, of the total amount (100%) of the unmodified antibody and modified antibody contained in the antibody composition. In addition, "containing a relatively large amount of divalent antibody" means that the amount of divalent antibody contained in the second antibody composition is larger than that of the monovalent antibody contained in the antibody composition, preferably that the amount of divalent antibody is not less than 55%, not less than 63%, not less than 70%, not less than 80%, or not less than 90%, of the total amount (100%) of the unmodified antibody and modified antibody contained in the antibody composition.

In the retention step, a solution containing the mixture of the unmodified antibody, monovalent antibody and divalent antibody obtained in the antibody modification step is added to a column, and unmodified antibody and monovalent antibody retained on the carrier are retained on the column and the divalent antibody not retained on the carrier is allowed to pass through. The solution that passed through in the retention step constitutes a part of the second antibody composition. To facilitate retention of the unmodified antibody and monovalent antibody on the column and to prevent aggregation or denaturation of these, it is preferable to dilute the mixed solution of the peptide-modified antibody with an appropriate dilution solvent and add same to the column. The dilution solvent is not particularly limited as long as the peptide-modified antibody dissolves and does not easily aggregate or denature in the solvent, and water, saline, buffers such as sodium acetate buffer, ammonium acetate buffer, phosphate buffer, phosphate buffered saline, 2-amino-2-(hydroxymethyl)propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. When a buffer is used as a dilution solvent, the concentration of the buffering agent is not less than 10 mmol/L, preferably not less than 15 mmol/L, more preferably not less than 20 mmol/L as the lower limit, and not more than 1000 mmol/L, preferably not more than 500 mmol/L, more preferably not more than 100 mmol/L as the upper limit. In addition, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the washing step, the modified antibody remaining in the column is eluted from the column by using a wash solvent. The solution that passed through the column in the aforementioned retention step and the solution eluted from the column in the washing step contain a relatively large amount of the divalent antibody, and therefore, these can be combined and used as the second antibody composition.

The wash solvent is not particularly limited as long as the peptide-modified antibody dissolves and does not easily aggregate or denature in the solvent, and it is a buffer having appropriate pH buffering capacity, and buffers such as sodium acetate buffer, ammonium acetate buffer, phosphate buffer, phosphate buffered saline, 2-amino-2-(hydroxymethyl) propane-1,3-diol (Tris) buffer, 2-[4-(2-hydroxyethyl)-1-piperazinyl]-ethanesulfonic acid (HEPES) buffer and the like, and the like can be used. It is preferable to use any of the aforementioned buffers, more preferably sodium acetate buffer. The concentration of the buffering agent used as the wash solvent is not less than 20 mmol/L, preferably not less than 30 mmol/L as the lower limit, and not more than 200 mmol/L, preferably not more than 70 mmol/L as the upper limit.

The pH of the wash solvent is not less than 4.0, preferably not less than 4.5, more preferably not less than 4.8 as the lower limit, and not more than 7.4, preferably not more than 6.0, more preferably not more than 5.2 as the upper limit. Furthermore, to reduce non-specific binding of the divalent antibody and antibody-modification peptide to the column carrier, the elution solvent may contain an additive such as sodium chloride, potassium chloride and the like. The concentration of the additive contained in the elution solvent is not particularly limited and may be, for example, 0.15 mol/L.

In the elution step, the modified antibody retained on the carrier is eluted from the column by using an elution solvent. That is, the first antibody composition containing a relatively large amount of unmodified antibody and monovalent antibody is eluted from the column by using an elution solvent.

As the elution solvent, a buffer such as sodium acetate buffer, ammonium acetate buffer, citrate buffer and the like, and the like can be used. In addition, to reduce non-specific binding to the antibody-modification linker, unmodified antibody and modified antibody column carrier, the elution solvent may contain an additive such as sodium chloride, potassium chloride and the like. The concentration of the additive contained in the elution solvent is not particularly limited and may be, for example, 0.15 mol/L.

When the elution solvent contains a buffering agent, the concentration of the buffering agent is not less than 20 mmol/L, preferably not less than 30 mmol/L as the lower limit, and not more than 200 mmol/L, preferably not more than 70 mmol/L as the upper limit. In addition, to weaken the interaction between the unmodified antibody and monovalent antibody, and the immunoglobulin-binding protein, and to prevent denaturation and aggregation of the antibody, the pH of the elution solvent is not less than pH 3.0 as the lower limit, and not more than pH 4.2 as the upper limit.

The first antibody composition or the second antibody composition obtained in the antibody purification step may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferred, and the range of not less than 0.1 mL and not more than 10 mL is more preferred.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferred, and the range of not less than 1 μmol/L and not more than 100 μmol/L is more preferred, from the aspect of the yield of the desired chelate conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferred, and the range of not less than 10 min and not more than 20 hr is more preferred.

The obtained chelate conjugate may be used as it is or purified using a filtration filter, a membrane filter, a column filled with various fillers, chromatography or the like.

In the chelate conjugate produced by steps (A) and (B), a specific site of a humanized antibody that specifically

binds to MUC5AC (e.g., the lysine residue in the Fc region of antibody) is specifically modified with a chelating agent. This chelate conjugate comprises one or two molecules of the aforementioned chelating agent per one molecule of the antibody. The chelating agent site-specifically modifies the Fc region of the antibody of the present invention via a linker. The linker is constituted of a chelate linker that connects to a chelating agent, a first atomic group that connects to the linker, a second atomic group that can perform click reaction with the first atomic group, and an antibody-modification linker that connects to the second atomic group (including an antibody-modification peptide represented by the above-mentioned formula (i)). Therefore, the linker has a chemical structure derived from the first atomic group and the second atomic group. As such chemical structure, a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

[Chem. 14]

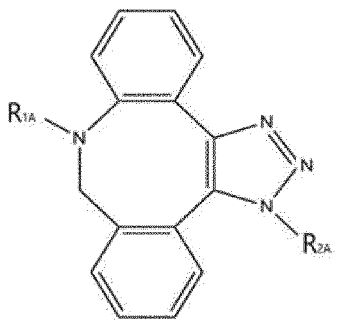

(10a)

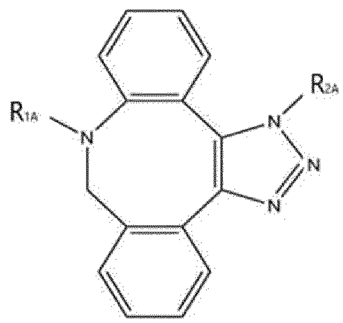

(10b)

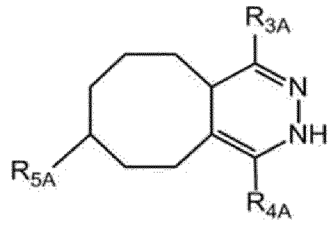

(10c)

In the formula (10a) and the formula (10b), $R_{1A}$ is a binding site with a chelate linker, and $R_{2A}$ is a binding site with an antibody-modification linker. In the formula (10c), one of $R_{3A}$ and $R_{4A}$ is a hydrogen atom, a methyl group, a phenyl group or a pyridyl group, and the other is a binding site with a chelate linker, and $R_{5A}$ is a binding site with an antibody-modification linker.

(1-5) Liquid pharmaceutical composition

The liquid pharmaceutical composition of the present invention refers to a pharmaceutical composition in a liquid form which contains the conjugate of the present invention, i.e., a conjugate of a radionuclide and an anti-MUC5AC specific antibody, and is in a form suitable for administration to the subject living body. As used herein, "liquid" refers to being a liquid or fluid state under general conditions (e.g., in the atmosphere, at room temperature/ordinary temperature), and also includes a state having some viscosity. Specific examples include, but are not limited to, solution, suspension, dispersion, emulsion, and the like. A liquid pharmaceutical composition can be produced, for example, by dissolving a conjugate of the present invention, which is produced by the aforementioned method, in a solvent mainly containing water and almost isotonic with the living body. In this case, the composition may contain other pharmaceutically acceptable components as necessary.

The concentration of the anti-MUC5AC specific antibody in the liquid pharmaceutical composition of the present invention is a concentration that can exert the desired effect, and is 0.1 mg/mL or more, preferably 0.3 mg/mL or more, more preferably 0.4 mg/mL or more, further preferably 0.5 mg/mL or more, and 5 mg/mL or less, preferably 3 mg/mL or less, more preferably 2 mg/mL or less, further preferably 1.5 mg/mL or less. In one embodiment of the present invention, the concentration of the anti-MUC5AC specific antibody is 0.1 mg/mL or more and 5 mg/mL or less, preferably 0.3 mg/mL or more and 3 mg/mL or less, more preferably 0.4 mg/mL or more and 2 mg/mL or less, further preferably 0.5 mg/mL or more and 1.5 mg/mL or less. Here, when the conjugate of the present invention is a conjugate of an antibody and a chelating agent with radionuclide chelated, the concentration of the above-mentioned anti-MUC5AC specific antibody is the total concentration of the antibody conjugated with the chelating agent and the antibody not conjugated with the chelating agent. Fig. 1 is a diagram for explaining the concentration of anti-MUC5AC specific antibody in the liquid pharmaceutical composition of the present invention. A is a schematic diagram of a chelate linker, B is a schematic diagram of a radionuclide, C is a schematic diagram of a radionuclide labeled chelator (intermediate) in which a radionuclide is complexed with the chelate linker of A, D is a schematic diagram of an antibody-modification peptide, E is a schematic diagram of an unmodified anti-MUC5AC specific antibody (zero-valent antibody), F is a schematic diagram of an anti-MUC5AC specific antibody (monovalent antibody) modified with one antibody-modification peptide molecule, G is a

schematic diagram of an anti-MUC5AC specific antibody (unlabeled monovalent antibody) in which an unlabeled chelate linker and an antibody-modification peptide are bound by a click reaction, and H is a schematic diagram of an anti-MUC5AC specific antibody in which a radionuclide-labeled chelate linker and an antibody-modification peptide are bound by a click reaction. When the active ingredient of the liquid pharmaceutical composition of the present invention has a structure represented by H, the concentration of the anti-MUC5AC specific antibody in the liquid pharmaceutical composition of the present invention is the total concentration of the zero-valent antibody (unmodified antibody), monovalent antibody, unlabeled monovalent antibody, and the active ingredient anti-MUC5AC specific antibody contained in the structure represented by H.

The concentration of the anti-MUC5AC specific antibody in the liquid pharmaceutical composition of the present invention is measured as a protein concentration immediately after production or during storage at 25±2°C, using a variable pathlength ultraviolet-visible spectrophotometer with a measurement wavelength of 280 nm set as the measurement conditions.

The liquid pharmaceutical composition of the present invention is characterized in that it contains a stabilizer and a buffering agent. The stabilizer may be amino acid, sugar, sugar alcohol, or inorganic salt. The amino acid as the stabilizer may be, for example, L-arginine or glycine. The sugar as the stabilizer is, for example, sucrose or trehalose. The sugar alcohol as the stabilizer is, for example, sorbitol. The inorganic salt as the stabilizer is, for example, sodium chloride. The buffering agent is, for example, L-histidine, citric acid, or phosphoric acid. Of these, L-arginine is preferred as the stabilizer, L-histidine is preferred as the buffering agent, and it is more preferred to use L-arginine and L-histidine in combination.

Each amino acid contained in the liquid pharmaceutical composition may be in the form of a salt. The salt form includes an acid addition salt and a salt with a base, and it is preferable to select a pharmacologically acceptable salt. Examples of such salts include salts with inorganic acids, salts with organic acids, salts with inorganic bases, and salts with organic bases. Examples of the salts with inorganic acids include salts with hydrohalic acids (hydrochloric acid, hydrobromic acid, hydroiodic acid, etc.), sulfuric acid, nitric acid, phosphoric acid, and the like. Examples of the salts with organic acids include salts with formic acid, acetic acid, propionic acid, oxalic acid, succinic acid, maleic acid, fumaric acid, citric acid, and the like. Examples of the salts with inorganic bases include salts with alkali metals such as sodium, potassium, and lithium, salts with alkaline earth metals such as calcium and magnesium, and salts with ammonium. Examples of the salts with organic bases include salts with ethylenediamine, propylenediamine, ethanolamine, monoalkyl ethanolamine, dialkyl ethanolamine, diethanolamine, triethanolamine and the like.

The concentration of each amino acid in the liquid pharmaceutical composition is appropriately determined depending on the type of amino acid to be used (when the amino acid is a salt, it is converted to the free form). When the stabilizer is L-arginine, it is generally 10 mmol/L or more, 25 mmol/L or more, or 50 mmol/L or more, more preferably 80 mmol/L or more, further preferably 90 mmol/L or more, and preferably 150 mmol/L or less, 140 mmol/L or less, or 130 mmol/L or less, more preferably 120 mmol/L or less, further preferably 110 mmol/L or less. In one embodiment of the present invention, the concentration of L-arginine is 10 mmol/L or more and 150 mmol/L or less, 25 mmol/L or more and 140 mmol/L or less, 50 mmol/L or more and 130 mmol/L or less, preferably 80 mmol/L or more and 120 mmol/L or less, more preferably 90 mmol/L or more and 110 mmol/L or less. When the buffering agent is L-histidine, it is preferably 1 mmol/L or more, 10 mmol/L or more or 20 mmol/L or more, more preferably 30 mmol/L or more, further preferably 40 mmol/L or more, and preferably 100 mmol/L or less, 90 mmol/L or less or 80 mmol/L or less, more preferably 70 mmol/L or less, further preferably 60 mmol/L or less. In one embodiment of the present invention, the concentration of L-histidine is 1 mmol/L or more and 100 mmol/L or less, or 10 mmol/L or more and 90 mmol/L or less, or 20 mmol/L or more and 80 mmol/L or less, preferably 30 mmol/L or more and 70 mmol/L or less, more preferably 40 mmol/L or more and 60 mmol/L or less. These amino acids optionally have the function as buffering agents.

The liquid pharmaceutical composition of the present invention is characterized in that it contains a surfactant. The "surfactant" as used in the present invention is defined to include any surfactant having a hydrophilic region and a hydrophobic region, which includes nonionic, cationic, anionic, and amphoteric surfactants. Preferred surfactants can be selected from, for example, polysorbate 20 (polyoxyethylene sorbitan monolaurate, Tween20, CAS number:9005-64-5), polysorbate 60 (polyoxyethylene sorbitan monostearate, Tween60, CAS number:9005-67-8), polysorbate 65 (polyoxyethylene sorbitan tristearate, Tween65, CAS number:9005-71-4), polysorbate 80 (polyoxyethylene sorbitan oleate, Tween80, CAS number:9005-65-6), poloxamer (e.g., poloxamer 188), and the like. Polysorbate 80 is preferred for the liquid pharmaceutical composition of the present invention. The surfactant may be present in the liquid pharmaceutical composition of the present invention at a concentration that varies depending on the type of surfactant to be used. It can be present at a concentration of generally 0.001 vol% or more, preferably 0.01 vol% or more, more preferably 0.03 vol% or more, further preferably 0.05 vol% or more, and generally 2.0 vol% or less, preferably 1.0 vol% or less, more preferably 0.5 vol% or less, further preferably 0.3 vol% or less, further more preferably 0.1 vol% or less.

In one embodiment of the present invention, the surfactant can be present in the liquid pharmaceutical composition of the present invention at a concentration of 0.001 vol% or more and 2.0 vol% or less, preferably 0.01 vol% or more and 1.0 vol% or less, more preferably 0.03 vol% or more and 0.5 vol% or less, further preferably 0.05 vol% or more and 0.3 vol% or less, further more preferably 0.05 vol% or more and 0.1 vol% or less.

The pH of the liquid pharmaceutical composition of the present invention is preferably 3 or more, more preferably 5 or more, and is preferably 9 or less, more preferably 8 or less. In one embodiment, the pH of the liquid pharmaceutical composition of the present invention is 3 or more and 9 or less, preferably 5 or more and 8 or less.

An effective amount of the liquid pharmaceutical composition of the present invention is orally or parenterally such as intravenously, subcutaneously, intraperitoneally, intramuscularly, or the like, administered to a living body, and is used for treatment of a disease, diagnosis of a disease, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is cancer. The cancer to be treated or diagnosed in the present invention includes, for example, pancreatic cancer, thyroid cancer, liver cancer, colorectal cancer, gastric cancer, esophageal cancer, lung cancer, urothelial cancer, breast cancer, cervical cancer, uterine cancer, ovarian cancer, bile duct cancer, biliary tract cancer, and endometrial carcinoma, and particularly, application to pancreatic cancer is preferred.

As used herein, the "effective amount" is an amount that can afford useful therapeutic effects in a subject of administration. The effective amount to be administered to a subject varies depending on the type of subject, body weight of the subject, dosage form (injection, etc.) and route (oral administration, parenteral administration, etc.) of administration, severity of disease (e.g., cancer), and the like. Physicians and veterinarians can consider these factors and determine the appropriate effective amount.

By selecting a radionuclide having a therapeutic effect, specifically $\alpha$ particle emitting nuclide, the liquid pharmaceutical composition of the present invention can be used for internal radionuclide therapy. In internal radionuclide therapy, a radiopharmaceutical is administered intravenously or orally, this radiopharmaceutical is accumulated in a lesion site such as a primary cancer lesion or a metastatic lesion, and the cancer cells in the lesion site are destroyed by radiation emitted from the radiopharmaceutical. Therefore, the liquid pharmaceutical composition of the present invention can be preferably used for internal radionuclide therapy of cancer. In this case, the administration amount and dosage of the pharmaceutical composition are appropriately selected according to the kind of the radionuclide, the effectiveness of the active ingredient, the form and route of administration, the stage of progression of the disease (particularly cancer), body shape, body weight, age of the patient, and the kind and amount of other therapeutic agent to be used in combination for the disease.

Furthermore, as the administration amount and dosage of the pharmaceutical composition, a human equivalent dose (HED), which is calculated by converting the dose for a mouse to a human based on the body surface area, can be used. HED is determined by the following formula.

$$\text{HED} = \text{animal dose in MBq/kg} \times (\text{animal weight in kg/human weight in kg})^{0.33}$$

For example, when the HED for a human weighing 60 kg is calculated using the dose for a mouse weighing 20 g and when the radionuclide is iodine-131, it can generally be administered at not more than 100 MBq/kg per dose. The effect can be exhibited even at a dose of not more than 50 MBq/kg one time. For example, when the radionuclide is yttrium-90, it can generally be administered at not more than 50 MBq/kg per dose. The effect can be exhibited even at a dose of not more than 30 MBq/kg one time. For example, when radionuclide is lutetium-177, it can generally be administered at not more than 50 MBq/kg per dose. The effect can be exhibited even at a dose of not more than 40 MBq/kg one time. Such dosage is an example of one embodiment of the present invention, and those skilled in the art can make appropriate conversion according to the above-mentioned formula, based on the body weights of mouse and human.

When radionuclide is iodine-131, the toxicity to the thyroid gland, where iodine accumulates physiologically, can be reduced by pre-administration of a non-radioactive iodine preparation (stable iodine preparation). As a stable iodine preparation, potassium iodide, potassium iodate, and the like can be used.

In addition, as another embodiment of the present invention, when a radionuclide ($^{68}Ga$, $^{64}Cu$, $^{86}Y$, $^{89}Zr$, $^{111}In$, $^{123}I$, $^{125}I$, $Al^{18}F$) that emits positron or gamma ray from a $\beta$-ray emitting nuclide is used as the radionuclide in the aforementioned conjugate, the obtained radioactive pharmaceutical composition may be used for cancer diagnosis in internal radionuclide therapy for cancer. The diagnostic pharmaceutical composition for cancer of the present invention may be used for diagnosis before performing internal radionuclide therapy for cancer, or may be used for diagnosis after performing an internal radionuclide therapy for cancer. Using the radiopharmaceutical for diagnosis before conducting an internal radionuclide therapy for cancer, selection of treatment can be performed as to whether or not to perform an internal radionuclide therapy for cancer using the liquid pharmaceutical composition of the present invention provided with a radionuclide that emits $\beta$-rays. Using the composition for diagnosis after conducting an internal radionuclide therapy for cancer, moreover, whether or not cancer using the liquid pharmaceutical composition of the present invention provided with a radionuclide that emits $\beta$-rays is effective can be judged, and a treatment plan including an increase or a decrease of dose and the like can be optimized.

The material of the storage container in which the liquid pharmaceutical composition of the present invention is

contained may be glass or plastic. The shape of the storage container in which the liquid pharmaceutical composition of the present invention is contained may be a vial or a syringe. It may be colorless or colored.

According to the embodiment of the present invention described above, in a radioactive liquid pharmaceutical composition containing a radioactive anti-MUC5AC specific antibody, since adsorption of radioactivity to a storage container is suppressed, the initial quality can be ensured.

When the liquid pharmaceutical composition of the present invention is contained in a storage container (preferably a glass container), the rate of radioactivity adsorbed to the storage container (container adsorption rate) varies depending on the type of radionuclide used. However, it is preferably 10% or less, more preferably 8% or less, and further preferably 6% or less.

The above-mentioned embodiment of the present invention includes the following technical ideas.

[1] A liquid pharmaceutical composition containing a conjugate of a radionuclide and antibody as an active ingredient, wherein

the aforementioned antibody is an antibody that specifically binds to mucin subtype 5AC,
a concentration of the aforementioned antibody in the aforementioned composition is 0.1 mg/mL or more and 5 mg/mL or less, and
the composition contains a stabilizer, a buffering agent, and a surfactant.

[2] The pharmaceutical composition of [1], wherein the aforementioned radionuclide is a radionuclide that emits $\alpha$ particle, positron, $\beta$-ray, or $\gamma$-ray.
[3] The pharmaceutical composition of [1] or [2], wherein the aforementioned stabilizer is L-arginine.
[4] The pharmaceutical composition of any of [1] to [3], wherein the aforementioned buffering agent is L-histidine.
[5] The pharmaceutical composition of any of [1] to [4], wherein the aforementioned stabilizer in the aforementioned composition is L-arginine, the aforementioned buffering agent is L-histidine,

a concentration of the aforementioned L-arginine is 10 mmol/L or more and 150 mmol/L or less, and
a concentration of the aforementioned L-histidine is 1 mmol/L or more and 100 mmol/L or less.

[6] The pharmaceutical composition of any of [1] to [5], wherein the aforementioned surfactant is at least one selected from the group consisting of polysorbate 20, polysorbate 60, polysorbate 65, and polysorbate 80.
[7] The pharmaceutical composition of any of [1] to [6], wherein a concentration of the aforementioned surfactant in the aforementioned composition is 0.01 vol% or more and 2.0 vol% or less.
[8] The pharmaceutical composition of any of [1] to [7], wherein a concentration of the aforementioned surfactant in the aforementioned composition is 0.01 vol% or more and 0.5 vol% or less.
[9] The pharmaceutical composition of any of [1] to [8], wherein the aforementioned radionuclide is $^{89}Zr$.
[10] The pharmaceutical composition of any of [1] to [8], wherein the aforementioned radionuclide is $^{225}Ac$.
[11] The pharmaceutical composition of any of [1] to [10], wherein the aforementioned antibody is a humanized antibody.[11-1] The pharmaceutical composition of [11], wherein the aforementioned humanized antibody is a humanized antibody having a heavy chain variable region consisting of

(1) the amino acid sequence shown in SEQ ID NO: 1 (H01),
(2) the amino acid sequence shown in SEQ ID NO: 2 (H02),
(3) the amino acid sequence shown in SEQ ID NO: 3 (H03), or
(4) the amino acid sequence shown in SEQ ID NO: 4 (H04), and a light chain variable region consisting of
(5) the amino acid sequence shown in SEQ ID NO: 5 (L01),
(6) the amino acid sequence shown in SEQ ID NO: 6 (L02),
(7) the amino acid sequence shown in SEQ ID NO: 7 (L03), or
(8) the amino acid sequence shown in SEQ ID NO: 8 (L04).

[11-2] The conjugate of [11], wherein the aforementioned humanized antibody is a humanized antibody having a heavy chain variable region consisting of

(1) the amino acid sequence shown in SEQ ID NO: 1 (H01), and a light chain variable region consisting of
(7) the amino acid sequence shown in SEQ ID NO: 7 (L03).

[12] The pharmaceutical composition of any of [1] to [11], [11-1], and [11-2], wherein the aforementioned conjugate is a conjugate of the aforementioned chelating agent with radionuclide chelated and an antibody.

[12-1] The pharmaceutical composition of [12], wherein the chelating agent site-specifically modifies an Fc region of the antibody via a linker.
[12-2] The pharmaceutical composition of [12] and [12-1], wherein the aforementioned linker comprises a peptide represented by the above-mentioned formula (i) which consists of not less than 13 and not more than 17 amino acid residues, and which is formed by a crosslinking reaction of the aforementioned peptide modified by a crosslinking agent and the aforementioned antibody.
[12-3] The pharmaceutical composition of [12], [12-1] and [12-2], wherein the aforementioned chelating agent has a structure derived from a compound represented by the above-mentioned formula (A) or a salt thereof.
[12-4] The pharmaceutical composition of any of [12], [12-1], [12-2], and [12-3], wherein the aforementioned linker comprises

a chelating linker connected to the aforementioned chelating agent,
a first atomic group connected to the chelating linker,
a second atomic group capable of click reaction with the aforementioned first atomic group, and
an antibody-modification linker connected to the aforementioned second atomic group and comprising the aforementioned peptide, and
the aforementioned linker includes any one of the chemical structures represented by the above-mentioned formulas (10a), (10b), and (10c) as a chemical structure derived from the aforementioned first atomic group and the aforementioned second atomic group.

[13] The pharmaceutical composition of any of [12], [12-1], [12-2], [12-3], and [12-4], wherein one molecule of the aforementioned chelating agent is site-specifically conjugated with one molecule of the aforementioned antibody.
[14] The pharmaceutical composition of any of [12], [13], [12-1], [12-2], [12-3], and [12-4], wherein a concentration of the aforementioned antibody in the aforementioned composition is a total concentration of the antibody conjugated with the aforementioned chelating agent and an antibody not conjugated with the aforementioned chelating agent.
[15] The pharmaceutical composition of [14], wherein a concentration of the aforementioned antibody in the aforementioned composition is 0.4 mg/mL or more and 2.0 mg/mL or less.
[16] The pharmaceutical composition of claim [14] or [15], wherein a concentration of the aforementioned antibody in the aforementioned composition is 0.5 mg/mL or more and 1.5 mg/mL or less.
[17] The pharmaceutical composition of any of [1] to [16], [11-1], [11-2], [12-1], [12-2], [12-3], and [12-4], which has a pH of 5 or more and 8 or less.
[18] The pharmaceutical composition of any of [1] to [17], [11-1], [11-2], [12-1], [12-2], [12-3], and [12-4], wherein an adsorption rate of the aforementioned radionuclide in the aforementioned composition to a container is 10% or less.

[Example]

The present invention is described in detail in the following with reference to Example, but the present invention is not limited to these.

Production Example 1: Production of anti-MUC5AC humanized antibody

In the same manner as in Production Example 1 described in WO 2021/075544, an anti-MUC5AC humanized antibody (antibody 1:H01L03) was obtained.
As used herein, H01 is the heavy chain variable region shown in SEQ ID NO: 1, and L03 is the light chain variable region shown in SEQ ID NO: 7. The antibody used in the following examples consists of heavy chain constant region 1 (SEQ ID NO: 23) and light chain constant region 1 (SEQ ID NO: 24), and a combination of the heavy chain variable region and light chain variable region of antibody 1 described above.

Production Example 2: Site-specific antibody modification with peptide linker

(1) Antibody modification step

A solution containing a peptide-modified antibody was obtained in the same manner as in WO 2021/075544, Production Example 2, Step (1) except that the reaction time between the peptide and the anti-MUC5AC humanized antibody produced in Production Example 1 was changed to 60 minutes.

(2) Peptide-modified antibody separation step

This peptide-modified antibody was diluted with 20 mmol/L sodium acetate buffer (pH 6.0), added to a Protein A column (manufactured by GE Healthcare, HiTrap MabSelect SuRe), and 0.05 mol/L sodium acetate buffer (pH 5.7) containing 0.15 mol/L sodium chloride was flown to recover the peptide-modified antibody modified with two peptide molecules. Thereafter, 0.05 mol/L sodium acetate buffer (pH 4.2) containing 0.15 mol/L sodium chloride was flown through the solution, and the peptide-modified antibody (monovalent antibody) modified with one peptide molecule was collected in a container to which 1 mol/L sodium acetate buffer (pH 6.0) had been added in advance (monovalent antibody recovery amount 38.6 mg, unmodified antibody:monovalent antibody:bivalent antibody ratio measured by hydrophobic column chromatography = 33.4:66.6:0.0). Each of the collected fractions was buffer-substituted with 50 mmol/L histidine buffer (pH 6.1) containing 0.1 mol/L arginine, by using an ultrafiltration filter (manufactured by Merck, model number: UFC903096).

The hydrophobic column chromatography conditions are as follows.

column: BioPro HIC HT (4.6 mm×10 cm, 2.3 μm)
detector: ultraviolet absorption spectrophotometer

(measurement wavelength: 280 nm)
mobile phase A: 50 mmol/L phosphate buffer (pH 7.0) containing 1.5 mol/L ammonium sulfate
mobile phase B: 50 mmol/L phosphate buffer (pH 7.0)
mobile phase C: water
feeding of mobile phase: The mixing ratio of mobile phase A, mobile phase B, and mobile phase C is changed as follows to control the concentration gradient.

[Table 1]

| time after injection (min) | mobile phase A (vol%) | mobile phase B (vol%) | mobile phase C (vol%) |
|---|---|---|---|
| 0 - 5.0 | 80 | 20 | 0 |
| 5.0 - 25.0 | 80→0 | 20→100 | 0 |
| 25.0 - 25.5 | 0 | 100→0 | 0→100 |
| 25.5 - 30.0 | 0 | 0 | 100 |
| 30.0 - 30.5 | 0→80 | 0→20 | 100→0 |
| 30.5 - 40.0 | 80 | 20 | 0 |
| flow rate: 0.6 mL per minute | | | |

Example 1: Production of $^{89}Zr$-labeled anti-MUC5AC specific antibody preparation

(1) Complex formation step

The structure of the chelating moiety (DOTA-GA-DBCO) used in this Example is shown in the following formula (L1-5). A $^{89}Y$ target was irradiated with protons and dissolved in a 0.1 mol/L aqueous hydrochloric acid solution to prepare a $^{89}Zr$ ion-containing solution (radioactivity concentration: 11286 MBq/mL, liquid volume: 0.140 mL). The solvent was distilled off under heating conditions. The chelating moiety was dispersed in 195 mmol/L acetic acid-sodium acetate buffer (pH 5.5) to obtain a dispersion containing 1.0 mmol/L of the chelating moiety (chelating moiety dispersion). 0.024 mL of this dispersion, 0.080 mL of 0.1 mol/L hydrochloric acid, 0.080 mL of 150 mmol/L gentisic acid solution (dissolved in 195 mmol/L acetic acid-sodium acetate buffer (pH 5.5)), and 0.056 mL of 195 mmol/L acetic acid-sodium acetate buffer (pH 5.5) were added to the radioactive metal source from which the solvent had been distilled off, and they were reacted under heating conditions to obtain a $^{89}Zr$ complex solution. The molar ratio of the chelating moiety to the radioactive metal ion was chelating moiety:$^{89}Zr$ ion = about 22.5:1, the heating temperature of the reaction mixture was 70°C, and the heating time was 90 min.

[Chem. 15]

L1-5

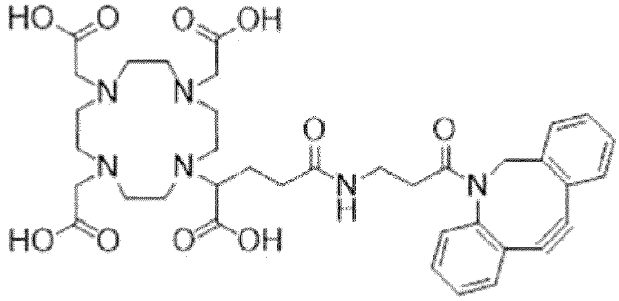

DOTA-GA-DBCO

(2) Labeling step

A solution of the $^{89}$Zr complex obtained through the aforementioned Step (1) was mixed with a solution containing the peptide-modified antibody (monovalent antibody) produced through Step (1) and Step (2) of Production Example 2, and a click reaction was performed at 37°C for 1.5 hr to obtain a $^{89}$Zr-labeled anti-MUC5AC specific antibody.

(3) Purification step

A solution containing the $^{89}$Zr-labeled anti-MUC5AC specific antibody obtained in the aforementioned Step (2) was dispensed into three ultrafiltration filters (Manufactured by Merck, model number: UFC505096), and the solvent was each replaced with 100 mmol/L arginine-containing 50 mmol/L histidine buffer (pH 5.1, 6.1, or 7.1) to obtain purified fractions. The radiochemical purity of the $^{89}$Zr-labeled anti-MUC5AC specific antibody after purification was 95%, and the radiochemical yield (no attenuation compensation) was 53%. The radiochemical purity and radiochemical yield of the $^{89}$Zr-labeled anti-MUC5AC specific antibody were measured as follows. That is, thin layer chromatography (manufactured by Agilent, model number: SGI0001, developing solvent: acetonitrile:0.1 mmol/L EDTA solution mixture (volume ratio 1:1)) was measured using a radio γ-TLC analyzer (manufactured by Raytest, MODEL GITA Star PS), and the percentage of the radioactivity (counts) of the peak detected near the point of origin relative to the total radioactivity (counts) detected was taken as the radiochemical purity (%). The radiochemical yield (%) was defined as the percentage of the radioactivity recovered after solvent replacement relative to the total radioactivity added at the start of the labeling step.

(4) Formulation step

To the purified fraction obtained in the aforementioned Step (3), in which the solvent was replaced with the 100 mmol/L arginine-containing 50 mmol/L histidine buffer, was added an appropriate amount of 0.7 w/v% polysorbate 80-containing 100 mmol/L arginine-containing 50 mmol/L histidine buffer (pH was 5.1, 6.1 or 7.1, the same solution was used) to formulate such that the concentration of polysorbate 80 in the preparation was 0.07 w/v%, and 4 mL thereof was filled into colorless glass vials (volume 6 mL) equipped with a rubber stopper. The radioactivity concentration in each dispensed vial immediately after production was 63.8 - 68.1 MBq/mL. Regarding the concentration of anti-MUC5AC specific antibody, the preparations with a target value of 0.5 mg/mL showed actual measured values of 0.43 to 0.45 mg/mL, and the preparations with a target value of 1.0 mg/mL showed actual measured values of 0.66 to 0.68 mg/mL.

The concentration of anti-MUC5AC specific antibody in the solution was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.46.

Example 2: Production of $^{89}$Zr-labeled anti-MUC5AC specific antibody preparation

After performing Step (1) and Step (2) of Example 1, the following steps were performed. The radioactivity concentration used in Step (1) was 7512 MBq.

(3) Purification step

After cooling the solution containing the $^{89}$Zr-labeled anti-MUC5AC specific antibody obtained in Step (2) to near room temperature, 4.0 mL of 20 mmol/L histidine buffer (pH 5.5) was added and the mixture was passed through a column (HiTrap SP FF 5 mL) packed with a cation exchange resin to retain the $^{89}$Zr-labeled anti-MUC5AC specific antibody in the column. Thereafter, 21 mL of 20 mmol/L histidine buffer (pH 5.5) was passed through the column for washing. 45 mL of 100 mmol/L arginine-containing, 50 mmol/L histidine buffer (pH 6.1) was passed through this column and combined with 5.0 mL of 240 mmol/L arginine-containing, 50 mmol/L histidine buffer (pH 6.1) containing 0.7 w/v% polysorbate 80 to obtain a solution of $^{89}$Zr-labeled anti-MUC5AC specific antibody (4938 MBq, 44 mL).

The radiochemical purity of the obtained $^{89}$Zr-labeled anti-MUC5AC specific antibody was 97%, and the radiochemical yield (no attenuation compensation) was 66%. The radiochemical purity and radiochemical yield of the $^{89}$Zr-labeled anti-MUC5AC specific antibody were measured in the same manner as in Example 1.

(4) Formulation step

The solution was filtered through a polyvinylidene fluoride membrane filter with a pore size of 0.22 μm, and an appropriate amount of the purified fraction obtained in the aforementioned Step (3) was added to a colorless glass vial (volume 6 mL) equipped with a rubber stopper to give a preparation shown by the following formulation.

The concentration of anti-MUC5AC specific antibody in the preparation was 1.15 mg/mL immediately after production. The concentration of anti-MUC5AC specific antibody in the solution was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.51.

| | |
|---|---|
| $^{89}$Zr-labeled anti-MUC5AC specific antibody (after completion of production) | 471 MBq |
| polysorbate 80 | 2.8 mg |
| L-arginine hydrochloride | 84.3 mg |
| L-histidine | 31.0 mg |
| water for injection | appropriate amount |
| hydrochloric acid | appropriate amount |
| total | 4.2 mL |

Example 3: Production of $^{225}$Ac-labeled anti-MUC5AC specific antibody

(1) Chelate modification step

In this example, the same chelating moiety as that shown in the above-mentioned formula (L1-5) was used. The chelating moiety was dispersed in 0.1 mol/L acetic acid-sodium acetate buffer (pH 5.0) as a solvent to obtain a dispersion containing 0.3 mmol/L chelating moiety. After irradiating a $^{226}$Ra target with protons, a mixture of a $^{225}$Ac ion-containing solution (0.1 mol/L aqueous hydrochloric acid solution, radioactivity concentration 5.1 MBq/mL, liquid volume 0.333 mL) of 1.7 MBq (calculated value by attenuation calculation from the radioactivity on the date of the test) obtained by purification by the method described in WO 2022/014555, 0.360 mL of this dispersion, and 0.540 mL of 0.1 mol/L acetic acid-sodium acetate buffer (pH 5.0) was reacted under heating conditions to obtain a $^{225}$Ac complex solution. The molar ratio of the chelating moiety to the radioactive metal ion was chelating moiety:$^{225}$Ac ion = about 46330:1, the heating temperature of the reaction mixture was 70°C, and the heating time was 15 min.

(2) Labeling step

A solution of the $^{225}$Ac complex obtained through the aforementioned Step (1) was mixed with a solution containing the peptide-modified antibody (monovalent antibody) produced through Step (1) and Step (2) of Production Example 2, and a click reaction was performed at 37°C for 2 hr to obtain a $^{225}$Ac-labeled anti-MUC5AC specific antibody.

(3) Purification step

A solution containing the $^{225}$Ac-labeled anti-MUC5AC specific antibody obtained in Step (2) was fed into a column (manufactured by Cytiva, HiTrap SP FF 5 mL) packed with a cation exchange resin. After 10 mL of 20 mmol/L histidine buffer (pH 5.5) was fed into the column, 18 mL of 50 mmol/L histidine buffer (pH 6.1) containing 100 mmol/L

arginine was fed, and the purified fraction was recovered in a vial to which 2 mL of 50 mmol/L histidine buffer (pH 6.1) containing 240 mmol/L arginine and 0.7 w/v% polysorbate 80 had previously been added. The radiochemical purity of the obtained $^{225}$Ac-labeled anti-MUC5AC specific antibody was 99%, and the radiochemical yield was about 40%. The radiochemical purity and radiochemical yield of the $^{225}$Ac-labeled anti-MUC5AC specific antibody were measured in the same manner as in Example 1.

(4) Formulation step

An appropriate amount of the purified fraction obtained in the aforementioned Step (3) was added to a colorless glass vial (volume 6 mL) equipped with a rubber stopper to give a preparation. The produced liquid pharmaceutical composition of the present invention containing the $^{225}$Ac-labeled anti-MUC5AC specific antibody as an active ingredient has the following formulation.

The concentration of anti-MUC5AC specific antibody in the preparation was 0.87 mg/mL immediately after production. The concentration of anti-MUC5AC specific antibody in the solution was measured by size-exclusion chromatography. The measurement conditions of the size-exclusion chromatography are as follows.
column: TSK gel UP-SW3000 (4.6×150 mm, 2.0 μm)
detector: ultraviolet absorption spectrophotometer (measurement wavelength: 280 nm)
mobile phase :0.2 mol/L arginine hydrochloride-containing 0.1 mol/L phosphate buffer (pH 6.8)
flow rate: 0.3 mL per minute

| | |
|---|---|
| $^{225}$Ac-labeled anti-MUC5AC specific antibody (produced in Step (2) above) | 37kBq |
| polysorbate 80 | 2.8 mg |
| L-arginine hydrochloride | 84.3 mg |
| L-histidine | 31.0 mg |
| water for injection | appropriate amount |
| hydrochloric acid | appropriate amount |
| total | 4 mL |

Production Example 3: Production of unlabeled chelate-modified anti-MUC5AC specific antibody (unlabeled conjugate) preparation

(1) Conjugate production step

The chelating moiety dispersion obtained in step (1) of Example 1 was used to perform a click reaction with the peptide-modified antibody in the same manner as in Step (2) of Example 1 to obtain an unlabeled antibody (unlabeled conjugate).

(2) Purification step

A solution containing an unlabeled chelate modified anti-MUC5AC specific antibody obtained by reacting at 37°C for 1.5 hr was dispensed into three ultrafiltration filters (Manufactured by Merck, model number: UFC505096), and the solvent was replaced with 100 mmol/L arginine-containing 50 mmol/L histidine buffer (pH 6.1) to obtain purified fractions.

(3) Formulation step

The purified fraction obtained in the aforementioned Step (2), an appropriate amount of 100 mmol/L arginine-containing 50 mmol/L histidine buffer (pH 6.1), and an appropriate amount of 0.7 w/v% polysorbate 80-containing 100 mmol/L arginine-containing 50 mmol/L histidine buffer (pH 6.1) were mixed, and an appropriate amount of polysorbate 80 was added such that the concentration of polysorbate 80 in the preparation was 0.07 w/v%. After passing through a membrane filter (manufactured by Merck, model number:SLGV033NB), the mixture was formulated by dispensing into colorless glass vials (volume 6 mL) equipped with a rubber stopper by 4 mL each.

The concentration of anti-MUC5AC specific antibody in the preparation was 1.0 mg/mL immediately after production. The concentration of anti-MUC5AC specific antibody in the solution was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.46.

Example 4: Production of $^{89}Zr$-labeled anti-MUC5AC specific antibody preparation

After performing Step (1) and Step (2) of Example 1 using radioactivity concentration 3880 MBq, Steps (3) and (4) of Example 2 were performed to produce a $^{89}Zr$-labeled anti-MUC5AC specific antibody preparation. The radiochemical purity of a solution (1890 MBq, 22 mL) of the $^{89}Zr$-labeled anti-MUC5AC specific antibody obtained in Step (3) was 91%, and the radiochemical yield (no attenuation compensation) was 49%. The concentration of anti-MUC5AC specific antibody in the preparation obtained in Step (4) was 0.96 mg/mL immediately after production, which was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.46. Other formulation was as shown in the following.

| | |
|---|---|
| $^{89}Zr$-labeled anti-MUC5AC specific antibody (after completion of production) | 122 MBq |
| polysorbate 80 | 1.4 mg |
| L-arginine hydrochloride | 42.2 mg |
| L-histidine | 15.5 mg |
| water for injection | appropriate amount |
| hydrochloric acid | appropriate amount |
| total | 2.0 mL |

Experimental Example 1: Evaluation of stability using anti-MUC5AC specific antibody and unlabeled conjugate of the present invention

Since it is not easy to evaluate the transport stability using radionuclide-labeled antibodies from the viewpoint of complying with the regulations for the transport of radioisotopes, etc., in vehicles, the transport stability (appearance, flow imaging, particle size measurement) of the unlabeled conjugate preparation obtained in Production Example 3 was evaluated as a preliminary test. In addition, the transport stability was similarly evaluated using an unmodified (intact) anti-MUC5AC specific antibody formulated using the anti-MUC5AC specific antibody obtained in Production Example 1 instead of the unlabeled conjugate.

Each formulation is shown in Table 2. In Table 2, PS80 is an abbreviation for polysorbate 80 (manufactured by NOF CORPORATION). Formulation 1-1 is a formulation of the unlabeled conjugate preparation of Production Example 3, and formulations 1-2, 1-3, and 1-4 are formulations of intact anti-MUC5AC specific antibodies (unmodified antibodies of Production Example 1). The concentration of unmodified antibodies in the solution was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.45.

[Table 2]

| formulation | | 1-1 | 1-2 | 1-3 | 1-4 |
|---|---|---|---|---|---|
| antibody | concentration | 1.0 mg/mL | | | |
| solvent | buffer | 100 mmol/L arginine-containing 50 mmol/L histidine buffer (pH 6.1) | | | |
| | additive | 0.07 w/v% PS80 | none | 0.07 w/v% PS80 | 0.7 w/v% PS80 |

The results of the appearance test (confirmation of the presence or absence of insoluble foreign matter by visual observation) after refrigerated storage (temperature range: 0.4 to 21.7°C) and land transportation (about 48 hr) are shown in Table 3, the results of the flow imaging are shown in Table 4 and Fig. 3, and the results of the particle size measurement are shown in Table 5.

As shown in Tables 3 and 4, the number of detected particles was mostly equivalent for formulation 1-1, formulation 1-3, and formulation 1-4, and the highest for Formulation 1-2.

Furthermore, as shown in Table 5, the numerical values of particle size and heterogeneity (%PD) were all within the normal range.

[Table 3]

| formulation | | 1-1 | 1-2 | 1-3 | 1-4 |
|---|---|---|---|---|---|
| results | before transportation | no foreign matter | | | |
| | after transportation | no foreign matter | many foreign matters | slight foreign matter | no foreign matter |

[Table 4]

| formulation | | 1-1 | 1-2 | 1-3 | 1-4 |
|---|---|---|---|---|---|
| results | before transportation → after transportation | almost perfectly spherical → air bubbles or oil | semitransparent amorphous form → aggregate | almost perfectly spherical → air bubbles or oil | almost perfectly spherical → air bubbles or oil |

[Table 5]

| formulation | | 1-1 | 1-2 | 1-3 | 1-4 |
|---|---|---|---|---|---|
| results | average particle size (nm) | 10.8-11.2 | 10.4-12.0 | 10.4-11.9 | 10.1-10.7 |
| | %PD | 14.4-18.8 | 10.6-23.5 | 10.8-19.1 | 6.6-15.7 |

Experimental Example 2: Effect of adding surfactant to solution containing $^{89}$Zr-labeled anti-MUC5AC specific antibody

Test samples were prepared based on the formulations shown in Table 6. Formulations 2-3, 2-5 to 2-7 are $^{89}$Zr-labeled anti-MUC5AC humanized antibodies formulated in Example 1, and formulations 2-1, 2-2, and 2-4 differ from the $^{89}$Zr-labeled anti-MUC5AC humanized antibody formulations produced in Example 1 in that they do not contain polysorbate 80.

[Table 6]

| formulation | | 2-1 | 2-2 | 2-3 | 2-4 |
|---|---|---|---|---|---|
| antibody | concentration | 0.5 mg/mL | | | |
| solvent | buffer | 100 mmol/L arginine-containing 50 mmol/L histidine buffer | | | |
| | pH | 5.1 | 6.1 | 6.1 | 7.1 |
| | surfactant | none | none | 0.07 w/v% PS80 | none |

| formulation | | 2-5 | 2-6 | 2-7 |
|---|---|---|---|---|
| antibody | concentration | 1.0 mg/mL | | |
| solvent | buffer | 100 mmol/L arginine-containing 50 mmol/L histidine buffer | | |
| | pH | 5.1 | 6.1 | 7.1 |
| | surfactant | 0.07 w/v% PS80 | 0.07 w/v% PS80 | 0.07 w/v% PS80 |

For each formulation, the storage condition was set at 25±2°C, and the degree of radioactivity adsorption to the storage container (container adsorption rate) was examined at three points of immediately after production (0-1 day after production), the midpoint (3-4 days after production), and the final point (6-7 days after production). The entire amount of sample solution was withdrawn from the storage container by using a micropipette. The liquid adhering to the rubber stopper was also removed using a micropipette. The radioactivity remaining in the storage container was measured using a radioisotope dose calibrator (model: CRC-15R, manufacturer: CAPINTEC). The container adsorption rate was calculated from the radioactivity [A] before withdrawal of the sample and the radioactivity [B] remaining in the storage container, by using the calculation formula shown by formula (1) and applying attenuation compensation (measurement time of [A] - measurement time of [B] = [C]). The unit of [C] is hour.

[Math. 1]

$$\text{container adsorption rate} = 100\times\frac{[B]\times 2^{(-([C]/78.41))}}{[A]} \quad (1)$$

The results of formulations 2-1 to 2-4 are shown in Fig. 2.

In Example 1, in which the surfactant polysorbate 80 was added (Formulation 2-3), adsorption of radioactivity to the container was greatly reduced.

Furthermore, the container adsorption rates for Formulations 2-5 to 2-7 were within the range of 1.7 to 2.1% throughout the storage period (Table 7).

[Table 7]

| formulation | results | |
|---|---|---|
| | 3 - 4 days after production | 6 - 7 days after production |
| 2-5 | 1.7 | 2.1 |
| 2-6 | 1.5 | 1.9 |
| 2-7 | 2.0 | 2.0 |

Experimental Example 3: Container adsorption rate of $^{89}$Zr-labeled anti-MUC5AC specific antibody preparation

The degree of radioactivity adsorption to the storage container (container adsorption rate) of the $^{89}$Zr-labeled anti-MUC5AC specific antibody preparation obtained in Example 2 was investigated. The measurement times were the starting point (0-2 days after production), the midpoint (5-6 days after production), and the final point (8-9 days after production), and the storage conditions were 5±3°C, 25±2°C, and 40±2°C, and the container adsorption rate was measured by a method similar to that in Experimental Example 2.

As a result, the container adsorption rate was within the range of 0.51-0.98% throughout the storage period under all storage conditions (Table 8).

[Table 8]

| storage temperature | 0 - 2 days after production | 5 - 6 days after production | 8 - 9 days after production |
|---|---|---|---|
| 5±3°C | 0.8 | 0.51 | 0.68 |
| 25±2°C | | 0.57 | 0.89 |
| 40±2°C | | 0.98 | 0.93 |

Experimental Example 4: Container adsorption rate of $^{225}$Ac-labeled anti-MUC5AC specific antibody preparation

The degree of radioactivity adsorption to the storage container of the solution produced in Example 3 (container adsorption rate) was examined. The measurement time was 4 days after production and the storage condition was 5±3°C, and the entire amount of sample solution was withdrawn from the storage container by using a micropipette. The liquid adhering to the rubber stopper was also removed using a micropipette. The radioactivity remaining in the storage container was measured using a gamma ray spectrometer (model: GMX10P4-70, manufacturer: ORTEC). The container adsorption rate was calculated from the radioactivity [A] before withdrawal of the sample and the radioactivity [B] remaining in the storage container, by using the following calculation formula and applying attenuation compensation (measurement time of [A] - measurement time of [B] = [C]). The unit of [C] is day.
[Math. 2]

$$\text{container adsorption rate} = 100\times\frac{[B]\times 2^{(-([C]/9.92))}}{[A]} \quad (2)$$

The container adsorption rate was 5.97%.

Experimental Example 5: Evaluation of stability of $^{89}Zr$-labeled anti-MUC5AC specific antibody preparation

The transport stability (appearance, particle size measurement) of the $^{89}Zr$-labeled anti-MUC5AC specific antibody preparation obtained in Example 4 was evaluated.

The concentration of unmodified antibodies in the solution was measured using a variable pathlength ultraviolet-visible spectrophotometer (SoloVPE, IN-VPE-Solo5, manufactured by C Technologies) with the measurement conditions set at a measurement wavelength of 280 nm and a molar absorption coefficient of 1.46. As a result, the antibody concentration was 0.96 mg/mL.

As a result of appearance test (confirmation of the presence or absence of insoluble foreign matter by visual observation) after refrigerated storage (temperature range: 4.8 to 24.1°C) and land and air transportation (about 48 hr), no foreign matter was found either before or after transportation. In addition, the particle size and non-uniformity (%PD) values before and after transportation were average particle size: 11.0 nm, %PD: 0.088 before transportation and average particle size: 10.8 nm, %PD: 0.159 after transportation, both of which were within the normal ranges.

This application is based on a patent application No. 2022-171832 filed in Japan (filing date: October 26, 2022), the contents of which are incorporated in full herein.

## Claims

1. A liquid pharmaceutical composition containing a conjugate of a radionuclide and antibody as an active ingredient, wherein

    the antibody is an antibody that specifically binds to mucin subtype 5AC,
    a concentration of the antibody in the composition is 0.1 mg/mL or more and 5 mg/mL or less, and
    the composition contains a stabilizer, a buffering agent, and a surfactant.

2. The pharmaceutical composition according to claim 1, wherein the radionuclide is a radionuclide that emits $\alpha$ particle, positron, $\beta$-ray, or $\gamma$-ray.

3. The pharmaceutical composition according to claim 1 or 2, wherein the stabilizer is L-arginine.

4. The pharmaceutical composition according to claim 1 or 2, wherein the buffering agent is L-histidine.

5. The pharmaceutical composition according to claim 1 or 2, wherein the stabilizer in the composition is L-arginine, the buffering agent is L-histidine,

    a concentration of the L-arginine is 10 mmol/L or more and 150 mmol/L or less, and
    a concentration of the L-histidine is 1 mmol/L or more and 100 mmol/L or less.

6. The pharmaceutical composition according to claim 1 or 2, wherein the surfactant is at least one selected from the group consisting of polysorbate 20, polysorbate 60, polysorbate 65, and polysorbate 80.

7. The pharmaceutical composition according to claim 1 or 2, wherein a concentration of the surfactant in the composition is 0.01 vol% or more and 2.0 vol% or less.

8. The pharmaceutical composition according to claim 1 or 2, wherein a concentration of the surfactant in the composition is 0.01 vol% or more and 0.5 vol% or less.

9. The pharmaceutical composition according to claim 1 or 2, wherein the radionuclide is $^{89}Zr$.

10. The pharmaceutical composition according to claim 1 or 2, wherein the radionuclide is $^{225}Ac$.

11. The pharmaceutical composition according to claim 1 or 2, wherein the antibody is a humanized antibody.

12. The pharmaceutical composition according to claim 1 or 2, wherein the conjugate is a conjugate of the chelating agent with radionuclide chelated and an antibody.

13. The pharmaceutical composition according to claim 12, wherein one molecule of the chelating agent is site-specifically conjugated with one molecule of the antibody.

**14.** The pharmaceutical composition according to claim 12, wherein a concentration of the antibody in the composition is a total concentration of the antibody conjugated with the chelating agent and an antibody not conjugated with the chelating agent.

**15.** The pharmaceutical composition according to claim 14, wherein a concentration of the antibody in the composition is 0.4 mg/mL or more and 2 mg/mL or less.

**16.** The pharmaceutical composition according to claim 14, wherein a concentration of the antibody in the composition is 0.5 mg/mL or more and 1.5 mg/mL or less.

**17.** The pharmaceutical composition according to claim 1, which has a pH of 5 or more and 8 or less.

[Fig. 1]

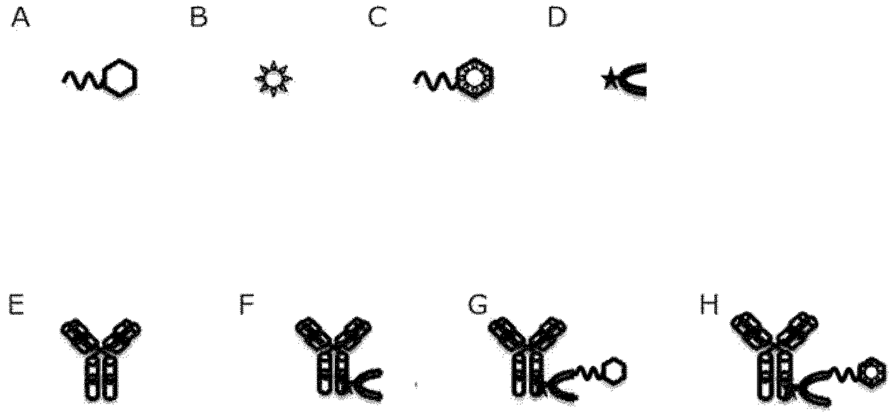
A
B
C
D
E
F
G
H

[Fig. 2]

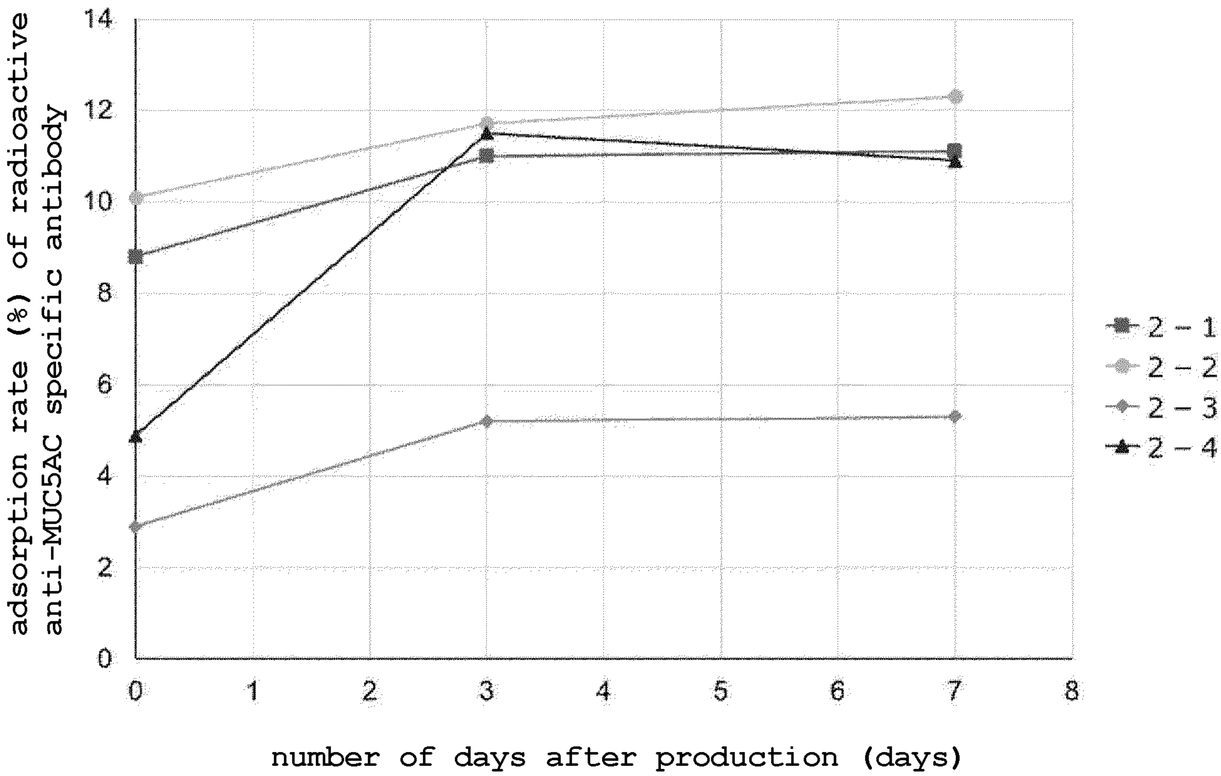
adsorption rate (%) of radioactive anti-MUC5AC specific antibody
14
12
10
8
6
4
2
0
0
1
2
3
4
5
6
7
8
number of days after production (days)
2 - 1
2 - 2
2 - 3
2 - 4

[Fig. 3]

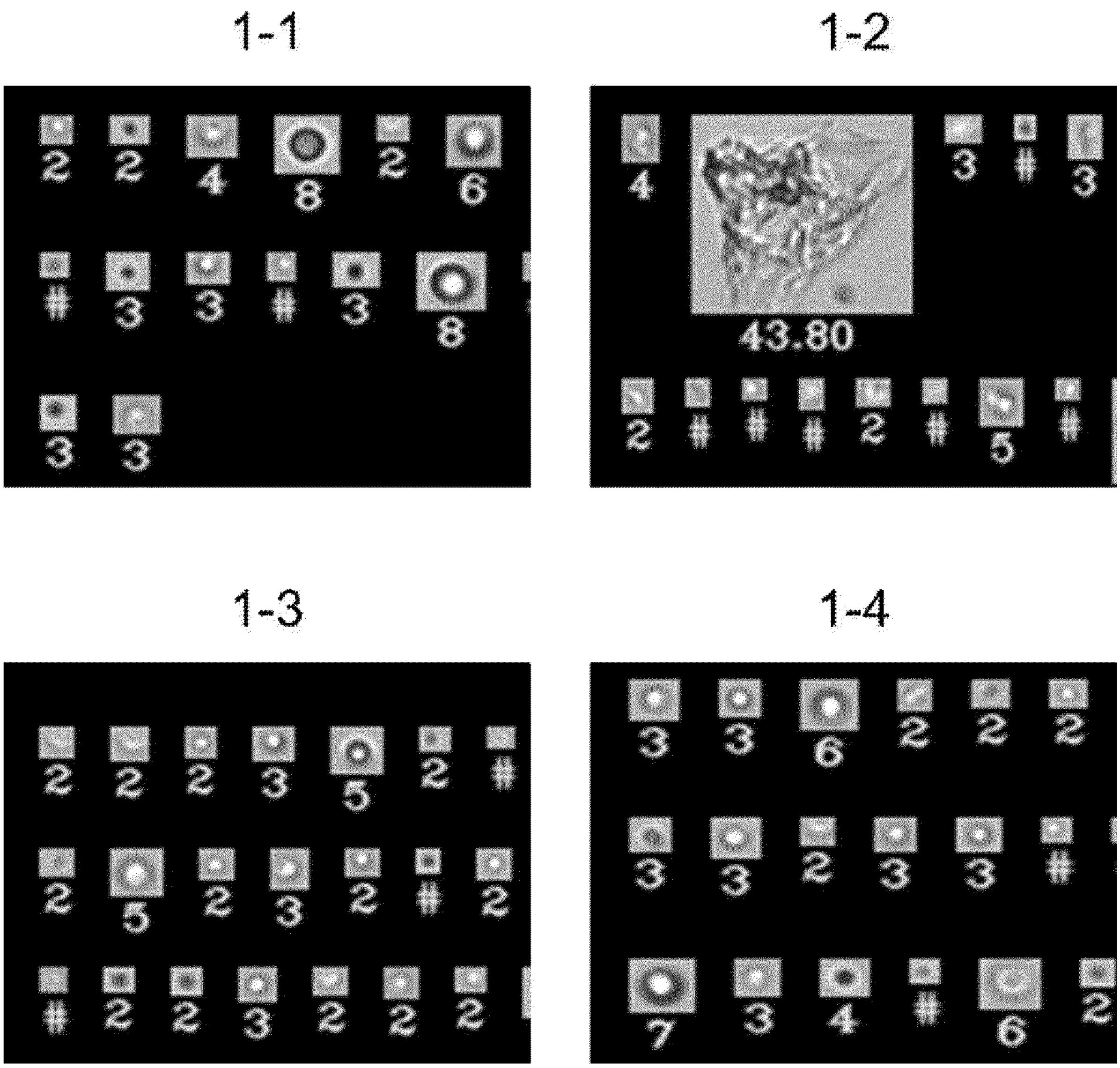

## INTERNATIONAL SEARCH REPORT

International application No. **PCT/JP2023/038580**

**A. CLASSIFICATION OF SUBJECT MATTER**

***A61K 47/68***(2017.01)i; ***A61K 9/08***(2006.01)i; ***A61K 39/395***(2006.01)i; ***A61K 47/18***(2017.01)i; ***A61K 47/26***(2006.01)i; ***A61K 51/10***(2006.01)i; ***A61P 35/00***(2006.01)i; ***C07K 16/18***(2006.01)i

FI: A61K47/68; A61K51/10 100; A61P35/00; A61K47/18; A61K47/26; A61K39/395 D; A61K9/08; C07K16/18; A61K39/395 N ZNA

According to International Patent Classification (IPC) or to both national classification and IPC

**B. FIELDS SEARCHED**

Minimum documentation searched (classification system followed by classification symbols)

A61K47/68; A61K9/08; A61K39/395; A61K47/18; A61K47/26; A61K51/10; A61P35/00; C07K16/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2021/75544 A1 (NIHON MEDI-PHYSICS CO., LTD.) 22 April 2021 (2021-04-22) claims, paragraphs [0110]-[0113], examples | 1-17 |
| Y | JP 2019-206559 A (CHUGAI PHARMACEUTICAL CO LTD) 05 December 2019 (2019-12-05) claims, examples | 1-17 |
| Y | JP 2018-30879 A (NOVO NORDISK AS) 01 March 2018 (2018-03-01) claims, examples | 1-17 |
| Y | JP 2012-519706 A (GENENTECH, INC.) 30 August 2012 (2012-08-30) claims, examples | 1-17 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/038580**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST. 26 format".

Form PCT/ISA/210 (continuation of first sheet) (January 2015)

**INTERNATIONAL SEARCH REPORT**
**Information on patent family members**

International application No.
**PCT/JP2023/038580**

| Patent document cited in search report | Publication date (day/month/year) | Patent family member(s) | Publication date (day/month/year) |
|---|---|---|---|
| WO 2021/75544 A1 | 22 April 2021 | US 2021/0338852 A1<br>claims, paragraphs [0157]-[0162], examples<br>EP 4049684 A4 | |
| JP 2019-206559 A | 05 December 2019 | US 2010/0285011 A1<br>claims, examples<br>EP 2238985 A1 | |
| JP 2018-30879 A | 01 March 2018 | US 2013/0136733 A1<br>claims, examples<br>EP 2575761 A1 | |
| JP 2012-519706 A | 30 August 2012 | US 2010/0239567 A1<br>claims, examples<br>EP 2403874 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H7203974 A
- JP H115749 A
- WO 2013157102 A
- WO 2013157105 A
- WO 2021075544 A
- JP H9510454 A
- JP 2003510368 A
- WO 2021075545 A
- WO 2016186206 A
- WO 2017217347 A
- WO 2018230257 A
- WO 2003080655 A
- WO 2011118699 A
- WO 2022014555 A
- JP 2022171832 A

### Non-patent literature cited in the description

- *Japanese Journal of Cancer Research*, 1996, vol. 87, 977-984
- *Nippon Rinsho*, 2006, vol. 64 (1), 274-278
- *Japanese Journal of Cancer Research*, 1999, vol. 90, 1179-1186
- *J Labelled Comp Radiopharm.*, 2014, vol. 57, 25-35
- *CHEMICAL ABSTRACTS*, 9005-64-5
- *CHEMICAL ABSTRACTS*, 9005-67-8
- *CHEMICAL ABSTRACTS*, 9005-71-4
- *CHEMICAL ABSTRACTS*, 9005-65-6